# EUROPEAN PATENT APPLICATION

(11) **EP 4 104 834 A1**
(43) Date of publication of application: **21.12.2022**
(21) Application number: 21179188.4
(22) Date of filing: 14.06.2021
(51) Int. Cl.: A61K 31/41, A61K 31/4745, A61P 35/00

(54) **COMBINATION TREATMENTS FOR CANCER PATIENTS AND METHODS FOR IDENTIFYING SAME**

(71) Applicant: Scandion Oncology A/S, 2100 Copenhagen Ø (DK)
(72) Inventor: Vestlev, Peter Michael, 2100 Copenhagen Ø (DK); Lindland Roest, Nicklas, 2100 Copenhagen Ø (DK)
(74) Representative: Høiberg P/S

(57) **Abstract**

The present invention relates to treatment of subgroups of cancer patients, methods for identifying treatment and dosage regimens, as well as methods for identifying these subgroups of cancer patients.

## Description

### Technical field

The present invention relates to treatment of subgroups of cancer patients, methods for identifying treatment and dosage regimens, as well as methods for identifying these subgroups of cancer patients.

### Background

Cancer is an overwhelming burden to our society with approximately 14 million new cases of cancer diagnosed in 2014. Despite the introduction of many new treatment modalities/options, *de novo* or acquired resistance to the applied treatments still represents the major cause of death from cancer.

Colorectal cancer (CRC) is one of the most common cancers in Europe and worldwide over 1.8 million new cases and 881,000 deaths are estimated to occur in 2018. Unfortunately, a large proportion of these patients will develop metastatic disease (mCRC) despite prior adjuvant treatment and approximately 20% of newly diagnosed CRC patients did present with metastatic disease before the introduction of screening. The standard of care to patients with local recurrence or mCRC is either surgery and/or chemotherapy and targeted therapy with monoclonal antibodies. For incurable patients, standard drugs are 5-flourouracil and derivatives, oxaliplatin, irinotecan, bevacizumab and panitumumab or cetuximab. The anti-cancer agent irinotecan is most often prescribed in combination with 5-flourouracil and leucovorin (FOLFIRI). One major problem in the treatment of mCRC is the frequent development of drug resistance. In practical terms, this means the cancer continues to either grow during anti-cancer treatment (de novo resistance) or re-grow after an initial response to anti-cancer treatment (acquired resistance). Acquired resistance is often accompanied by cross-resistance to other anti-cancer drugs.

Every year approximately 1000 Danish patients are diagnosed with Pancreatic Cancer (PC). Among the few approved treatments for inoperable PC is FOLFIRINOX which is a combination of irinotecan, 5-flourouracil and leucovorin in combination with oxaliplatin, or the combination of gemcitabine and nab-paclitaxel that was introduced in 2013 as a result of a large phase III study. Pancreatic cancer patients are fragile and only about 50% of the patients tolerate the treatments named above, the rest of the patients are treated with gemcitabine as monotherapy or best supportive care.

Pancreatic cancer has a high frequency of primary (de novo) resistance against chemotherapy, but also fast development of secondary (acquired) resistance is a great problem as most patients, who have an initial positive effect of chemotherapy, will experience disease progression at a later time-point. In both pancreatic cancer and metastatic colorectal cancer, which both are cancers with few treatment options, irinotecan is an important anti-cancer agent in the palliative phase of the illness.

Irinotecan is often used in the treatment of mCRC and pancreatic cancer. However, most of the patients rapidly develop resistance to this drug. Upregulation of the ABCG2 drug efflux pump is often observed in irinotecan resistant disease and since the active metabolite of irinotecan, SN-38, is a substrate for this efflux pump, ABCG2 upregulation is considered a major resistance mechanism against irinotecan treatment. Neutropenia and diarrhea are frequent dose-limiting side effects of irinotecan and may be severe. Pharmacokinetic variability has been related to biliary excretion of unconjugated bilirubin. Even modest elevations in serum bilirubin increase the risk for severe neutropenia and diarrhea associated with increased concentrations of SN-38. Patients with inherited genetic polymorphism in the drug-metabolizing enzyme UGT1A1*28, which metabolizes the active form of irinotecan, SN-38, may have more severe irinotecan-related neutropenia and diarrhea.

The compound SCO-101, also known as NS3728 was first described in WO 2000/24707. SCO-101 has later been shown to be an effective potentiator of a range of anti-cancer agents and is currently being developed for cancer combination therapies, in particular for treatment of resistant cancers. WO 2017/198700 describes SCO-101 and its use in combination therapies for treatment of cancers.

There is a need in the art for providing improved treatment regimens for cancer patients, in particular to overcome cancer resistance while minimizing common adverse events, such as neutropenia.

### Summary

The present inventors have found in a clinical study of cancer patients that following the last treatment with SCO-101, blood bilirubin concentrations were observed to normalize completely to baseline results indicating that the inhibition of UGT1A1 had ceased and that SCO-101's inhibition of UGT1A1 is completely reversible. The reversibility and that fact that SCO-101 is a potent inhibitor of UGT1A1 with an IC₅₀ of approximately 0.7 to 1.9 µM makes UGT1A1 inhibitors, including SCO-101, promising candidates for assessing patients' rate of bilirubin normalization (the bilirubin index) prior to initiation of treatment with anti-cancer agents known to be substrates for UGT1A1 and to adjust the dosage of the anti-cancer agent according to the bilirubin index.

Surprisingly, the present inventors have established a close correlation between the bilirubin index and the patients' RAS mutation status. As approximately 40-50% of mCRC patients and more than 90% of pancreatic cancer patients have cancers with RAS mutations, this correlation provides an important prediction model of which patients can tolerate treatment with anti-cancer agents that are UGT1A1 substrates, such as irinotecan/SN-38, and by dose adjustments enable treatment of the different subpopulations of cancer patients. The methods disclosed herein thus allow for identification of suitable treatment regimens for different patient groups and decrease or prevent the risk of serious side effects that may result in discontinuation of treatment. In addition, the present inventors have identified suitable treatment regimens for patients having a RAS mutated cancer, and patients having a cancer which is RAS wild-type.

Thus, in a first aspect, a method of treating a patient having cancer is provided comprising administering an UGT1A1 inhibitor and an anti-cancer agent to the patient; wherein the cancer is not a RAS mutated cancer.

In a second aspect, a method of treating a patient having cancer is provided comprising administering an UGT1A1 inhibitor and an anti-cancer agent to the patient; wherein the cancer is a RAS wild-type.

In a third aspect, a method of treating a patient having cancer is provided comprising administering an UGT1A1 inhibitor and an anti-cancer agent to the patient; wherein the cancer is a RAS mutated cancer.

In a fourth aspect, a method for identifying a treatment regimen is provided for a patient having cancer with an UGT1A1 inhibitor and an anti-cancer agent; said method comprising determining if the patient has a RAS mutated cancer; and wherein if the cancer is not a RAS mutated cancer, the dosage regimen is a first predefined dosage of the UGT1A1 inhibitor and a first predefined dosage of the anti-cancer agent; or
wherein if the cancer is a RAS mutated cancer, the dosage regimen is a second predefined dosage of the UGT1A1 inhibitor and a second predefined dosage of the anti-cancer agent;
wherein the first and second predefined dosages of the anti-cancer agent are different; and/or
wherein the first and second predefined dosages of the UGT1A1 inhibitor are different.

In a fifth aspect, a method for identifying a treatment regimen is provided for a patient having cancer and treating the patient with an UGT1A1 inhibitor and an anti-cancer agent; said method comprising determining if the patient has a RAS mutated cancer; and
administering a first predefined dosage of the UGT1A1 inhibitor and a first predefined dosage of the anti-cancer agent to the patient that does not have a RAS mutated cancer; or
administering a second predefined dosage of the UGT1A1 inhibitor and a second predefined dosage of the anti-cancer agent to the patient having a RAS mutated cancer;
wherein the first and second predefined dosages of the anti-cancer agent are different; and/or
wherein the first and second predefined dosages of the UGT1A1 inhibitor are different.

In a sixth aspect, a method is provided for selecting an anti-cancer agent dosage regimen for a cancer patient, wherein the anti-cancer agent is an UGT1A1 substrate; said method comprising the consecutive steps of:
1) administering a first predefined dosage of an UGT1A1 inhibitor to the patient one or more times;
2) determining the plasma concentration of unconjugated bilirubin in the patient at a first time point;
3) discontinuing administration of the UGT1A1 inhibitor;
4) determining the plasma concentration of unconjugated bilirubin at a second time point which is subsequent in time to the first time point;
5) generating a bilirubin index being the second plasma concentration of unconjugated bilirubin divided by the first plasma concentration of unconjugated bilirubin, and wherein if the bilirubin index is below a threshold value, selecting a first predefined dosage of the anti-cancer agent as dosage regimen; or
wherein if the bilirubin index is above a threshold value, selecting a second predefined dosage of the anti-cancer agent as dosage regimen;
wherein the first and second predefined dosages of the anti-cancer agent are different.

In a seventh aspect, a method for identifying an anti-cancer agent dosage regimen is provided for a cancer patient and treating the cancer patient, wherein the anti-cancer agent is an UGT1A1 substrate; said method comprising the consecutive steps of:
1) administering a first predefined dosage of an UGT1A1 inhibitor to the patient one or more times;
2) determining the plasma concentration of unconjugated bilirubin in the patient at a first time point;
3) discontinuing administration of the UGT1A1 inhibitor;
4) determining the plasma concentration of unconjugated bilirubin at a second time point which is subsequent in time to the first time point;
5) generating a bilirubin index being the second plasma concentration of unconjugated bilirubin divided by the first plasma concentration of unconjugated bilirubin, and
6) administering a first predefined dosage of the anti-cancer agent to the patient having a bilirubin index below a threshold value; or
7) administering a second predefined dosage of the anti-cancer agent to the patient having a bilirubin index above the threshold value;
wherein the first and second predefined dosages of the anti-cancer agent are different.

In an eighth aspect, a method for predicting if a patient is at risk of developing grade 3 or 4 neutropenia in response to an anti-cancer agent is provided, wherein the anti-cancer agent is an UGT1A1 substrate, said method comprising the consecutive steps of:
1) administering a first predefined dosage of an UGT1A1 inhibitor to the patient one or more times;
2) determining a first concentration of unconjugated bilirubin at a first time point;
3) discontinuing administration of the UGT1A1 inhibitor;
4) determining a second concentration of unconjugated bilirubin at a second time point which is subsequent in time to the first time point; and
wherein the patient is at risk of developing grade 3 or 4 neutropenia if the bilirubin index being the second concentration divided by the first concentration is above a threshold value; and/or
wherein the patient is not at risk of developing grade 3 or 4 neutropenia if the bilirubin index being the second concentration divided by the first concentration is below the threshold value.

In a ninth aspect, a method for predicting if a patient has or is likely to have a RAS-like mutated cancer is provided, wherein the anti-cancer agent is an UGT1A1 substrate, said method comprising the consecutive steps of:
1) administering a predefined dosage of an UGT1A1 inhibitor to the patient one or more times; and
2) determining a first concentration of unconjugated bilirubin at a first time point;
3) discontinuing treatment with the UGT1A1 inhibitor;
4) determining a second concentration of unconjugated bilirubin at a second time point which is subsequent in time to the first time point; and
wherein the patient has or is likely to have a RAS-like mutated cancer if the bilirubin index being the second concentration divided by the first concentration is above a threshold value; and/or
wherein the patient does not have or is not likely to have a RAS-like mutated cancer if the bilirubin index being the second concentration divided by the first concentration is below the threshold value.

In a tenth aspect, a combination drug is provided comprising,
a) separately or together, 0.3 mmol of an UGT1A1 inhibitor, and 50% of 180 mg/m² of an anti-cancer agent which is an UGT1A1 substrate; or
b) separately or together, 0.2 mmol of an UGT1A1 inhibitor, and 50% of 180 mg/m² of an anti-cancer agent which is an UGT1A1 substrate.

### Description of Drawings

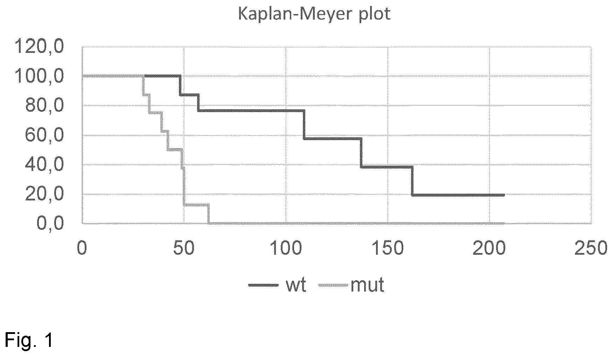

**Fig. 1****:** Kaplan-Meier plot of patients in cohort 1 having a cancer that is not RAS mutated, i.e. RAS wild-type (wt) compared to patients having a RAS mutated cancer (mut). The treatment comprises 150 mg SCO-101 and the dose of FOLFIRI varies between 65% and 89% of prior tolerated dose repeated every 14 days and a cohort of patients receiving 100 mg SCO-101 and a dose of FOLFIRI of 50% of the recommended dose. The patients having a cancer which is RAS wild-type tolerate the combination treatment of SCO-101 and FOLFIRI well and appear to have a significant benefit compared to patients having a RAS mutated cancer. The number of days from the start of the trial is shown on the X-axis, and the cumulative (number of patients in percentage) progression-free survival (PFS) is shown on the Y-axis.

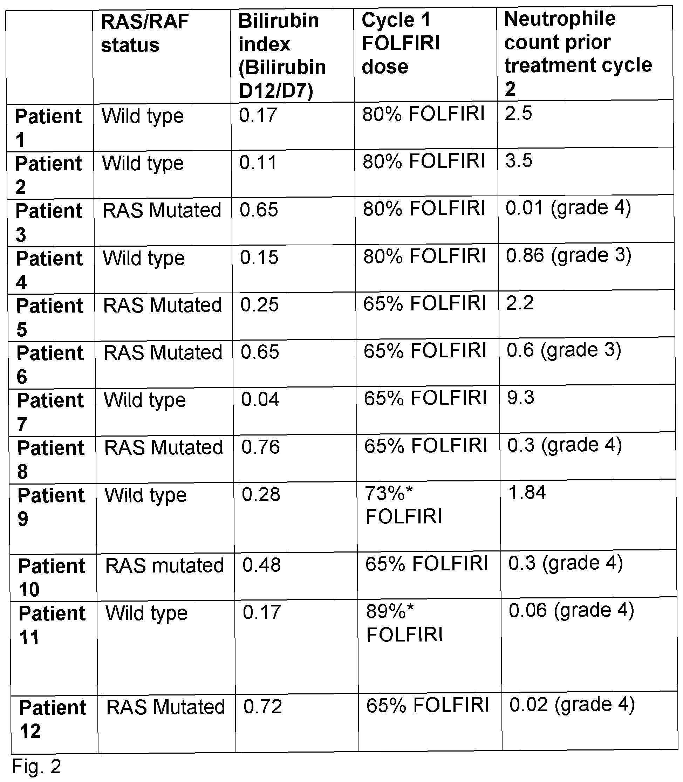

**Fig. 2****:** Patients stratified for RAS mutation status displaying information from the first treatment cycle with regards to bilirubin index value, FOLFIRI dose and corresponding neutrophile count prior to treatment in cycle 2. FOLFIRI dose is given in % of previous well tolerated (PWT) dose. The numbers followed by an asterisk (*) should have been 65% of the previously well tolerated (PWT) doses, but were incorrectly dosed as 65% of the recommended dose, leading to higher doses than planned. The bilirubin index is calculated by dividing day 12 with day 7 value for unconjugated (or total) bilirubin. A bilirubin index value higher than 0.30 is indicative of a low neutrophile count corresponding to dose limiting neutropenia. A neutrophile count of <1.0 is grade 3 neutropenia and <0.5 is grade 4. Grade 2 neutropenia is not considered dose limiting.

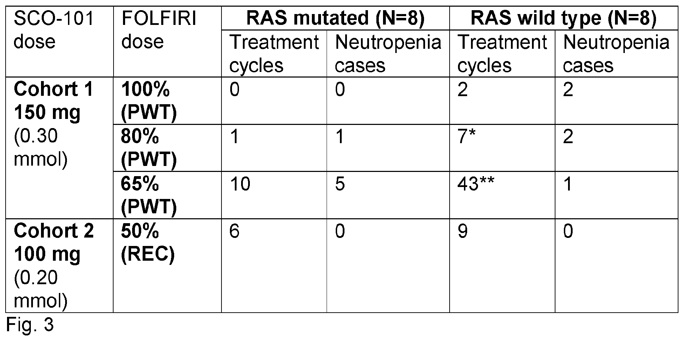

**Fig. 3****:** Number of treatment cycles completed (all cycles) and number of grade 3 or grade 4 events of neutropenia stratified by SCO-101 dose, FOLFIRI dose and RAS status. Dose of FOLFIRI is shown as either % of previous well tolerated dose during previous treatment (PWT) or % of recommended dose according to the Summary of Product Characteristics, SmPC (REC).

### Detailed description

### Definitions

The term "anti-cancer agent" as used herein includes, but is not limited to, a chemotherapeutic agent, that has activity against a susceptible tumour.

The term "RAS mutated cancer" as used herein refers to a cancer which is clinically classified as a cancer having one or more mutations in one or more RAS proto-oncogenes. The most common RAS proto-oncogenes are HRAS, NRAS and KRAS). Whether or not a cancer is a RAS mutated cancer can be determined clinically in ways known to the skilled person, such as by examining a tumour biopsy from the patient or in any other suitable way, such as by analysing RAS mutation status in isolated circulating tumour cells (CTCs). A patient suffering from a cancer that is classified as RAS mutated means that the cancer cells contain one or more RAS mutations that result in constitutively active RAS.

The term "RAS-like mutated cancer" as used herein refers to a cancer including, but not limited to, a RAS mutated cancer. The RAS-like mutated cancer covers a cancer having one or more mutations in one or more RAS proto-oncogenes, but also covers a cancer with a pathological behaviour similar to a RAS mutated cancer. A RAS-like mutated cancer would thus require clinical attention similar to a RAS mutated cancer. A RAS-like mutated cancer in a patient can be distinguished from a RAS wild-type cancer by determining the patient's rate of bilirubin normalization subsequent to administration of an UGT1A1 inhibitor to the patient, i.e. the "bilirubin index".

The term "bilirubin index" as used herein or "bilirubin index score" which are used interchangeably, refer to a patient's rate of bilirubin normalization subsequent to administration of an UGT1A1 inhibitor to the patient. Herein, the bilirubin index is determined subsequent to administration of an UGT1A1 inhibitor to the patient for a number of consecutive days, such as 1, 2, 3, 4, 5, or 6, preferably 6 days. Based on 6 consecutive days of administration with an UGT1A1 inhibitor, the bilirubin index is determined as the bilirubin serum concentration (unconjugated or total bilirubin) on day 12 divided with the bilirubin serum concentration on day 7 and thus is an indicator of how much the patients' UGT1A1 activity has normalized following cessation of SCO-101 treatment, and can be determined using bilirubin as a surrogate marker

The term "RAS wild-type" as used herein in the context of a cancer being a RAS wild-type refers to a cancer which is clinically classified as a cancer which does not have one or more mutations in one or more RAS proto-oncogenes. Whether or not a cancer is a RAS wild-type can be determined clinically in ways known to the skilled person, such as by examining a tumour biopsy from the patient or in any other suitable way, such as by analysing RAS mutation status in isolated circulating tumour cells (CTCs). A patient having a cancer which is RAS wild-type is thus not suffering from a cancer that is classified as RAS mutated.

The term "pharmaceutically acceptable salt" as used herein include without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

The term "FOLFIRI" used herein is a chemotherapy regimen for treatment of cancer, such as colorectal cancer. It is made up of the following drugs FOL - folinic acid (leucovorin), a vitamin B derivative used as a "rescue" drug for high doses of the drug methotrexate, but increases the cytotoxicity of 5-fluorouracil; F - fluorouracil (5-FU), a pyrimidine analog and antimetabolite which incorporates into the DNA molecule and stops synthesis; and IRI - irinotecan (Camptosar), a topoisomerase inhibitor, which prevents DNA from uncoiling and duplicating. One recommended dosage regimen consists of: irinotecan (180 mg/m² IV over 90 minutes) concurrently with folinic acid (400 mg/m² [or 2 x 250 mg/m²] IV over 120 minutes) followed by 5-fluorouracil (400-500 mg/m² IV bolus) then 5-fluorouracil (2400-3000 mg/m² intravenous infusion over 46 hours).

The term "treatment regimen" used herein refers to a method for treating cancer or tumours in a patient that includes administering simultaneously or sequentially a therapeutically effective amount of an UGT1A1 inhibitor and an anti-cancer agent.

The phrase "therapeutically effective amount" means that amount of such a substance, composition, kit or treatment regimen as a whole that produces some desired local or systemic effect, typically at a reasonable benefit/risk ratio in the context of a treatment regimen or method. The therapeutically effective amount of such substance will vary depending upon the patient and disease condition being treated, the weight and age of the patient, the severity of the disease condition, the manner of administration and the like. For example, certain compositions described herein may be administered in a sufficient amount to produce a desired effect at a reasonable benefit/risk ratio applicable to such treatment

The term "UGT1A1 inhibitor" as used herein refers to a molecule which is capable of binding to the enzyme uridine diphosphate (UDP)-glycuronosyl transferase (UGT1A1) and decrease the enzymatic activity of UGT1A1. The UGT1A1 enzyme, primarily found in the liver, is responsible for the gluronidation of bilirubin, converting it from the toxic form of bilirubin (unconjugated bilirubin) to its nontoxic, water-soluble form (conjugated bilirubin). Whether or not a particular molecule is an UGT1A1 inhibitor can be determined by examining the molecule's IC₅₀ *in vitro* in common UGT1A1 inhibition assays. An UGT1A1 inhibitor according to the present disclosure has an IC₅₀ of 20 µM or less, such as 15 µM or less, such as 10 µM or less, such as 5 µM or less. In one embodiment, the IC₅₀ is 5 µM or less, such as 4 µM or less, such as 3 µM or less, such as 2 µM or less, for example between 0.1 µM and 2.0 µM. In a preferred embodiment, the UGT1A1 inhibitor is SCO-101 with an IC₅₀ of approximately 0.7 to 1.9 µM. In some embodiments, the UGT1A1 inhibitor of the present disclosure is a non-competitive UGT1A1 inhibitor. In some embodiments, the UGT1A1 inhibitor is a competitive UGT1A1 inhibitor. In one embodiment, the UGT1A1 inhibitor is also SRPK1 inhibitor and/or an ABCG2 inhibitor.

The term "UGT1A1 substrate" as used herein refers to a molecule which is capable of being chemically transformed by enzymatic aid of uridine diphosphate (UDP)-glycuronosyl transferase (UGT1A1). Typically, the UGT1A1 substrate is capable of being glucuronidated by UGT1A1. In one embodiment, the UGT1A1 substrate is also an anti-cancer agent. In one embodiment, the UGT1A1 substrate is irinotecan, or its active metabolite SN-38.

The term "recommended dose" as used herein refers to the recommended dosage or dose of the anti-cancer agent as approved by the medicines agency, such as the EMA, the FDA or the Danish Medicines Agency. The doses of the anti-cancer agent irinotecan (CAMPTOSAR) recommended by the FDA are:
- Colorectal cancer combination regimen 1: CAMPTOSAR 125 mg/m² intravenous infusion over 90 minutes on days 1, 8,15, 22 with LV 20 mg/m² intravenous bolus infusion on days 1, 8, 15, 22 followed by 5-FU intravenous bolus infusion on days 1, 8, 15, 22 every 6 weeks.
- Colorectal cancer combination regimen 2: CAMPTOSAR 180 mg/m² intravenous infusion over 90 minutes on days 1, 15, 29 with LV 200 mg/m2 intravenous infusion over 2 hours on days 1, 2, 15, 16, 29, 30 followed by 5-FU 400 mg/m2 intravenous bolus infusion on days 1, 2, 15, 16, 29, 30 and 5-FU 600 mg/m2 intravenous infusion over 22 hours on days 1, 2, 15, 16, 29, 30.
- Colorectal cancer single agent regimen 1: CAMPTOSAR 125 mg/m2 intravenous infusion over 90 minutes on days 1, 8, 15, 22 then 2-week rest. (2.2).
- Colorectal cancer single agent regimen 2: CAMPTOSAR 350 mg/m2 intravenous infusion over 90 minutes on day 1 every 3 weeks. (2.2)

The skilled person will know, based on medicines agencies' recommendations as described above, which dose of an anti-cancer agent is the recommended dose.

### Treatment of subgroups of cancer patients

### Cancer which is not RAS-mutated

The present disclosure provides a method of treating a patient having cancer comprising administering an UGT1A1 inhibitor and an anti-cancer agent to the patient; wherein the cancer is not a RAS mutated cancer. As demonstrated in Example 2, patients having a cancer which is not RAS mutated, tolerate the combination treatment of SCO-101 and FOLFIRI well and appear to have a significant benefit compared to patients having a RAS mutated cancer.

In this context, the patient having a cancer which is not a RAS-mutated cancer is not suffering from a cancer that is classified as RAS mutated. Further, the present disclosure provides a method of treating a patient having cancer comprising administering an UGT1A1 inhibitor and an anti-cancer agent to the patient; wherein the cancer is a RAS wild-type.

In one embodiment, a method of treating a patient having cancer is provided comprising administering a first predefined dosage of an UGT1A1 inhibitor and a first predefined dosage of an anti-cancer agent to the patient; wherein the cancer is a RAS wild-type.

In one embodiment, the first predefined dosage of the UGT1A1 inhibitor is higher compared to a second predefined dosage of the UGT1A1 inhibitor administered to a patient having a cancer which is RAS mutated.

In one embodiment, the first predefined dosage of the anti-cancer agent is higher compared to a second predefined dosage of the anti-cancer agent administered to a patient having a cancer which is RAS mutated.

In one embodiment, the first predefined dosage of the UGT1A1 inhibitor is 0.30 mmol.

In one embodiment, the first predefined dosage of the UGT1A1 inhibitor is 0.30 mmol, the anti-cancer agent is irinotecan; and the first predefined dosage of irinotecan is below 180 mg/m².

In one embodiment, the method comprises administering a dose of the UGT1A1 inhibitor to the patient having a cancer which is a RAS wild-type, wherein the dose of the UGT1A1 inhibitor is higher compared to the dose of UGT1A1 inhibitor administered to a patient having a cancer which is RAS mutated.

In one embodiment, a method of treating a patient having cancer is provided comprising administering a first predefined dosage of an UGT1A1 inhibitor and a first predefined dosage of an anti-cancer agent to the patient; wherein the cancer is not a RAS mutated cancer.

In one embodiment, the method comprises administering a dose of the anti-cancer agent to the patient having a cancer which is a RAS wild-type, wherein the dose of the anti-cancer agent is higher compared to the dose of anti-cancer agent administered to a patient having a cancer which is RAS mutated.

In one embodiment, the method comprises administering a dose of the UGT1A1 inhibitor to the patient having a cancer which is a RAS wild-type, wherein the dose of the UGT1A1 inhibitor is higher compared to the dose of UGT1A1 inhibitor administered to a patient having a cancer which is RAS mutated and wherein the dose of the anti-cancer agent is higher compared to the dose of anti-cancer agent administered to a patient having a cancer which is RAS mutated.

In one embodiment, the cancer does not have a RAS mutation resulting in constitutive activation of RAS in a gene selected from the group consisting of: KRAS, HRAS, and NRAS. In one embodiment, the cancer does not have a KRAS mutation.

In one embodiment, the method of treatment disclosed herein is provided, wherein the UGT1A1 inhibitor is administered to the patient in a daily dosage of from 0.1 mmol to 0.4 mmol, such as from 0.1 mmol to 0.2 mmol, such as from 0.2 mmol to 0.3 mmol, such as from 0.3 mmol to 0.4 mmol, for example 0.2 mmol, or for example 0.3 mmol. In one embodiment, the UGT1A1 inhibitor is administered to the patient in a daily dosage of 0.3 mmol. In one embodiment, the UGT1A1 inhibitor is SCO-101 which is administered to the patient in a daily dosage of 0.3 mmol corresponding to 150 mg.

In one embodiment, the method of treatment disclosed herein is provided, wherein the UGT1A1 inhibitor is administered to the patient in a daily dosage of from 0.2 mmol to 0.4 mmol, for example 0.3 mmol; and the anti-cancer agent is administered to the patient at from 40% to 60% of the recommended dose according to the Summary of Product Characteristics, such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60%.

In one embodiment, the method of treatment disclosed herein is provided, wherein the UGT1A1 inhibitor is administered to the patient in a daily dosage of from 0.2 mmol to 0.4 mmol, for example 0.3 mmol; and the anti-cancer agent is irinotecan which is administered to the patient at from 40% to 60% of 180 mg/m², such as 50% of 180 mg/m².

### RAS-mutated cancer

The present disclosure further provides a method of treating a patient having cancer comprising administering an UGT1A1 inhibitor and an anti-cancer agent to the patient; wherein the cancer is a RAS mutated cancer. As demonstrated in Example 3, patients with a RAS mutated cancer, require a lowered dosage of the combination treatment of SCO-101 and FOLFIRI compared to the patients having cancer without a RAS mutation, and further the patients with the RAS mutated cancer tolerate the lowered dosage well.

In one embodiment, a method of treating a patient having cancer is provided comprising administering a second predefined dosage of an UGT1A1 inhibitor and a second predefined dosage of an anti-cancer agent to the patient; wherein the cancer is a RAS mutated cancer.

In one embodiment, the second predefined dosage of the UGT1A1 inhibitor is lower compared to the first predefined dosage of the UGT1A1 inhibitor administered to a patient having a cancer which is not a RAS mutated cancer.

In one embodiment, the second predefined dosage of the anti-cancer agent is lower compared to the first predefined dosage of the anti-cancer agent administered to a patient having a cancer which is not a RAS mutated cancer. In one embodiment, the second predefined dosage of the UGT1A1 inhibitor is below 0.30 mmol. In one embodiment, the second predefined dosage of the anti-cancer agent is below 180 mg/m² irinotecan or an equimolar dose of any other anti-cancer agent. In one embodiment, the second predefined dosage of the UGT1A1 inhibitor is below 0.30 mmol, the anti-cancer agent is irinotecan; and the second predefined dosage of irinotecan is below 180 mg/m².

In one embodiment, the anti-cancer agent is administered to the patient at a dosage which is lower than the recommended dose according to the Summary of Product Characteristics. The Summary of Product Characteristics is issued by the medicines agency, such as the Danish Medicines Agency, the EMA, or the FDA.

In one embodiment, the anti-cancer agent is administered to the patient at a dosage which is lower than the previously well tolerated (PWT) dosage of the anti-cancer agent when administered to the patient without an UGT1A1 inhibitor. In one embodiment, the anti-cancer agent is irinotecan which is administered to the patient at a dosage which is lower than 180 mg/m².

In one embodiment, the method comprises administering a dose of the UGT1A1 inhibitor to the patient having a cancer which is a RAS mutated cancer, wherein the dose of the UGT1A1 inhibitor is lower compared to the dose of UGT1A1 inhibitor administered to a patient having a cancer which is a RAS wild-type.

In one embodiment, the method comprises administering a dose of the anti-cancer agent to the patient having a cancer which is a RAS mutated cancer, wherein the dose of the anti-cancer agent is lower compared to the dose of anti-cancer agent administered to a patient having a cancer which is RAS wild-type.

In one embodiment, the method comprises administering a dose of the UGT1A1 inhibitor to the patient having a cancer which is a RAS mutated cancer, wherein the dose of the UGT1A1 inhibitor is lower compared to the dose of UGT1A1 inhibitor administered to a patient having a cancer which is a RAS wild-type and wherein the dose of the anti-cancer agent is lower compared to the dose of anti-cancer agent administered to a patient having a cancer which is RAS wild-type.

In one embodiment, the method comprises treatment of a cancer having a RAS mutation resulting in constitutive activation of RAS in a gene selected from the group consisting of: KRAS, HRAS, and NRAS. In one embodiment, the cancer has a KRAS mutation.

In one embodiment, the method of treatment disclosed herein is provided, wherein the UGT1A1 inhibitor is administered to the patient in a daily dosage of from 0.1 mmol to 0.4 mmol, such as from 0.1 mmol to 0.2 mmol, such as from 0.2 mmol to 0.3 mmol, such as from 0.3 mmol to 0.4 mmol, for example 0.2 mmol, or for example 0.3 mmol. In one embodiment, the UGT1A1 inhibitor is administered to the patient in a daily dosage of 0.2 mmol. In one embodiment, the UGT1A1 inhibitor is SCO-101 which is administered to the patient in a daily dosage of 0.2 mmol corresponding to 100 mg.

### Particular treatment aspects

In one embodiment, the UGT1A1 inhibitor is SCO-101: 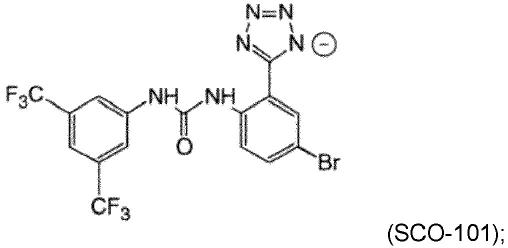 or a pharmaceutically acceptable salt thereof.

In one embodiment, the UGT1A1 inhibitor is administered to the patient daily. Daily administration may be administration once a day or more than once a day. In one embodiment, SCO-101 is administered to the patient daily.

In one embodiment, the method of treatment using the UGT1A1 inhibitor and the anti-cancer agent disclosed herein is provided, comprising a treatment cycle wherein the UGT1A1 inhibitor is administered to the patient daily from a first day to a sixth day, and the anti-cancer agent is administered to the patient from a fifth day to a seventh day.

In one embodiment, the treatment cycle can be repeated a number of times, such as 2 times or more, such as 3 times or more, such as 4 times or more, such as 5 times or more, such as 6 times or more, such as 7 times or more, such as 8 times or more, such as 9 times or more, such as 10 times or more, such as 11 times or more, such as 12 times or more, such as 13 times or more, such as 14 times or more, such as 15 times or more, such as 16 times or more, such as 17 times or more, such as 18 times or more, such as 19 times or more, such as 20 times or more, such as 21 times or more, such as 22 times or more, such as 23 times or more, such as 24 times or more, such as 25 times or more, such as 26 times or more, such as 27 times or more, such as 28 times or more, such as 29 times or more, such as 30 times or more, 31 times or more, such as 32 times or more, such as 33 times or more, such as 34 times or more, such as 35 times or more, such as 36 times or more, such as 37 times or more, such as 38 times or more, such as 39 times or more, such as 40 times or more, such as 41 times or more, such as 42 times or more, such as 43 times or more, such as 44 times or more, such as 45 times or more, such as 46 times or more, such as 47 times or more, such as 48 times or more, such as 49 times or more, such as 50 times or more, such as 51 times or more.

In one embodiment the treatment comprises administering one or more further agents, such as one or more further anti-cancer agents to the patient.

In one embodiment, the treatment further comprises administering to the patient a Granulocyte colony-stimulating factor (G-CSF). In one embodiment, the method further comprises administering to the patient a Granulocyte colony-stimulating factor (G-CSF) prior to or simultaneously with administration of the anti-cancer agent.

In one embodiment, the treatment further comprises administering to the patient a Granulocyte colony-stimulating factor (G-CSF) prior to or simultaneously with administration of the anti-cancer agent and subsequent to administration of the UGT1A1 inhibitor within a treatment cycle, optionally wherein G-CSF is administered one or more times during the treatment cycle.

### Anti-cancer agents

In one embodiment the anti-cancer agent is an UGT1A1 substrate.

In one embodiment, the anti-cancer agent is selected from the group consisting of: a topoisomerase inhibitor, an anti-hormone agent, an alkylating agent, a mitotic inhibitor, an antimetabolite, an anti-tumour antibiotic, a corticosteroid, a targeted anti-cancer therapy, a differentiating agent, and an immunotherapy.

In one embodiment, the anti-cancer agent is a topoisomerase inhibitor. In one embodiment, the anti-cancer agent is a topoisomerase I inhibitor or topoisomerase II inhibitor. In one embodiment, the anti-cancer agent is a topoisomerase I inhibitor selected from the group consisting of: irinotecan, its active metabolite SN-38, and topotecan.

In one embodiment, the anti-cancer agent is an anti-hormone agent. In one embodiment, the anti-cancer agent is an anti-estrogen, such as fulvestrant or tamoxifen.

In one embodiment, the anti-cancer agent is an alkylating agent, such as temozolomide.

In one embodiment, the anti-cancer agent is a mitotic inhibitor, such as paclitaxel or docetaxel.

In one embodiment, the anti-cancer agent is an antimetabolite, such as 5-fluorouracil (5-FU).

In one embodiment, the anti-cancer agent is a topoisomerase inhibitor. In one embodiment, the anti-cancer agent is a topoisomerase I inhibitor or topoisomerase II inhibitor. In one embodiment, the anti-cancer agent is a topoisomerase I inhibitor selected from the group consisting of: irinotecan, its active metabolite SN-38, and topotecan.

In one embodiment, the anti-cancer agent is administered in combination with a further anti-cancer agent. In one embodiment, the anti-cancer agent is administered in combination with a further anti-cancer agent which is 5-fluorouracil. In one embodiment, the anti-cancer agent is administered in combination with 5-fluorouracil and folinic acid. In one particular embodiment, the anti-cancer agent is irinotecan and is administered in combination with 5-fluorouracil and folinic acid (the combination of FOLFIRI).

### Cancers

In one embodiment, the method of treatment disclosed herein is provided for treatment of a cancer which is a solid tumour, such as a solid tumour selected from sarcoma, carcinoma and lymphoma.

In one embodiment, the treatment is provided for a cancer selected from the group consisting of Colorectal cancer, Breast cancer, lung cancer (non small cell lung cancer and small cell lung cancer), Glioblastomas, Head and neck cancers, Malignant melanomas, Basal cell skin cancer, Squamous cell skin cancer, Liver cancer, Pancreatic cancer, Prostate cancer, anal cancer, Cervix uteri cancer, Bladder cancer, Corpus uteri cancer, Ovarian cancer, Gall bladder cancer, Sarcomas, Leukemia's (myeloid and lymphatic), Lymphomas, Myelomatosis, and cholangiocarcinoma.

Cholangiocarcinoma is also known as bile duct cancer.

In one embodiment, the cancer is metastatic cancer. In one embodiment, the cancer is colorectal cancer, such as metastatic colorectal cancer.

In one embodiment, the cancer is pancreatic cancer, such as metastatic pancreatic cancer.

In one embodiment, the cancer is breast cancer, such as metastatic breast cancer.

In one embodiment, the method of treatment disclosed herein is provided, wherein the cancer is a resistant cancer which is resistant to the anti-cancer agent when administered alone.

In one embodiment, the method of treatment disclosed herein is provided, wherein the resistance is *de novo* resistance. In one embodiment, the resistance is acquired resistance.

In one embodiment, treatment with the UGT1A1 inhibitor re-sensitises the cancer to the anti-cancer agent.

### Method for identifying treatment or dosage regimen

### Regimen according to RAS status

As demonstrated by Example 4, examining the RAS mutation status prior to initiating treatment using SCO-101 and an anti-cancer agent, allows for identification of an improved treatment regimen prior to treatment. Thus, a method employing a predetermination of RAS mutation status prior to treatment can improve the course of treatment for patients about to receive combination therapy by limiting the risk of severe side effects, e.g. neutropenia, and the need for later dose adjustments and associated risk of treatment delays and disease progression.

The present disclosure further provides a method for identifying a treatment regimen for a patient having cancer with an UGT1A1 inhibitor and an anti-cancer agent; said method comprising determining if the patient has a RAS mutated cancer; and wherein if the cancer is not a RAS mutated cancer, the dosage regimen is a first predefined dosage of the UGT1A1 inhibitor and a first predefined dosage of the anti-cancer agent; or
wherein if the cancer is a RAS mutated cancer, the dosage regimen is a second predefined dosage of the UGT1A1 inhibitor and a second predefined dosage of the anti-cancer agent;
wherein the first and second predefined dosages of the anti-cancer agent are different; and/or
wherein the first and second predefined dosages of the UGT1A1 inhibitor are different.

In one embodiment, the method of identifying a treatment regimen further includes administering the identified treatment regimen to the patient.

Thus, the present disclosure further provides a method for identifying a treatment regimen for a patient having cancer and treating the patient with an UGT1A1 inhibitor and an anti-cancer agent; said method comprising determining if the patient has a RAS mutated cancer; and
administering a first predefined dosage of the UGT1A1 inhibitor and a first predefined dosage of the anti-cancer agent to the patient that does not have a RAS mutated cancer; or
administering a second predefined dosage of the UGT1A1 inhibitor and a second predefined dosage of the anti-cancer agent to the patient having a RAS mutated cancer;
wherein the first and second predefined dosages of the anti-cancer agent are different; and/or
wherein the first and second predefined dosages of the UGT1A1 inhibitor are different.

In one embodiment, the first and second predefined dosages of the anti-cancer agent are the same; and the first and second predefined dosages of the UGT1A1 inhibitor are different.

In one embodiment, the first and second predefined dosages of the anti-cancer agent are the same; and the first predefined dosage of the UGT1A1 inhibitor is higher than the second predefined dosage of the UGT1A1 inhibitor.

In one embodiment, the first and second predefined dosages of the UGT1A1 inhibitor are the same; and the first and second predefined dosages of the anti-cancer agent are different.

In one embodiment, the first and second predefined dosages of the UGT1A1 inhibitor are the same; and the first predefined dosage of the anti-cancer agent is higher than the second predefined dosage of the anti-cancer agent.

### Regimen according to bilirubin index

As demonstrated by Example 5, administering SCO-101 prior to initiating cancer therapy, and measuring the rate of unconjugated bilirubin normalization i.e. the bilirubin index, can provide a more precise anti-cancer agent dosage which is less likely to induce side effects. Thus, a method employing a pre-administration of SCO-101 followed by determination of the bilirubin index prior to administration of anti-cancer agents can improve the course of treatment for patients about to receive cancer therapy with an UGT1A1 substrate in combination with an UGT1A1 inhibitor such as SCO-101.

The present disclosure further provides a method is provided for selecting an anti-cancer agent dosage regimen for a cancer patient, wherein the anti-cancer agent is an UGT1A1 substrate; said method comprising the consecutive steps of:
1) administering a first predefined dosage of an UGT1A1 inhibitor to the patient one or more times;
2) determining the plasma concentration of unconjugated bilirubin in the patient at a first time point;
3) discontinuing administration of the UGT1A1 inhibitor;
4) determining the plasma concentration of unconjugated bilirubin at a second time point which is subsequent in time to the first time point;
5) generating a bilirubin index being the second plasma concentration of unconjugated bilirubin divided by the first plasma concentration of unconjugated bilirubin, and wherein if the bilirubin index is below a threshold value, selecting a first predefined dosage of the anti-cancer agent as dosage regimen; or
wherein if the bilirubin index is above a threshold value, selecting a second predefined dosage of the anti-cancer agent as dosage regimen;
wherein the first and second predefined dosages of the anti-cancer agent are different.

In a further embodiment, a method for identifying an anti-cancer agent dosage regimen is provided for a cancer patient and treating the cancer patient, wherein the anti-cancer agent is an UGT1A1 substrate; said method comprising the consecutive steps of:
1) administering a first predefined dosage of an UGT1A1 inhibitor to the patient one or more times;
2) determining the plasma concentration of unconjugated bilirubin in the patient at a first time point;
3) discontinuing administration of the UGT1A1 inhibitor;
4) determining the plasma concentration of unconjugated bilirubin at a second time point which is subsequent in time to the first time point;
5) generating a bilirubin index being the second plasma concentration of unconjugated bilirubin divided by the first plasma concentration of unconjugated bilirubin, and
6) administering a first predefined dosage of the anti-cancer agent to the patient having a bilirubin index below a threshold value; or
7) administering a second predefined dosage of the anti-cancer agent to the patient having a bilirubin index above the threshold value;
wherein the first and second predefined dosages of the anti-cancer agent are different.

In one embodiment, no UGT1A1 inhibitor is administered to the patient between steps 2) and 4). In one embodiment, the second time point is 5 days subsequent to the first time point.

In one embodiment, the threshold value is from 0.3 to 0.5, such as from 0.3 to 0.4, such as from 0.4 to 0.5, for example 0.4. In one embodiment, the threshold value is 0.3. In one embodiment, the threshold value is 0.5.

### Predefined dosages

Based on the methods for identifying a treatment or dosage regimen as described herein, predefined dosages can be identified.

In one embodiment, the second predefined dosage of the anti-cancer agent is lower than the first predefined dosage of the anti-cancer agent. In one embodiment, the second predefined dosage of the UGT1A1 inhibitor is lower than the first predefined dosage of the UGT1A1 inhibitor. In one embodiment, the first predefined dosage of the anti-cancer agent and the second predefined dosage of the anti-cancer agent are the same, and the second predefined dosage of the UGT1A1 inhibitor is lower than the first predefined dosage of the UGT1A1 inhibitor. In one embodiment, the first predefined dosage of the anti-cancer agent and the second predefined dosage of the anti-cancer agent are the same, and the second predefined dosage of the UGT1A1 inhibitor is from 50 to 70% lower than the first predefined dosage of the UGT1A1 inhibitor, such as 60% lower.

In one embodiment, the first predefined dosage of the UGT1A1 inhibitor is from 0.1 mmol to 0.4 mmol, such as from 0.1 mmol to 0.2 mmol, such as from 0.2 mmol til 0.3 mmol, such as from 0.3 mmol to 0.4 mmol.

In one embodiment, the first predefined dosage of the anti-cancer agent is from 40% to 60% of the recommended dose according to the Summary of Product Characteristics, such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60%.

In one embodiment, the first predefined dosage of the anti-cancer agent is from 40% to 60% of 180 mg/m², such as 50% of 180 mg/m².

In one embodiment, the first predefined dosage of the UGT1A1 inhibitor is from 0.1 mmol to 0.4 mmol, such as from 0.1 mmol to 0.2 mmol, such as from 0.2 mmol til 0.3 mmol, such as from 0.3 mmol to 0.4 mmol.

In one embodiment, the UGT1A1 inhibitor is administered to the patient in a daily dosage of from 0.2 mmol to 0.4 mmol, for example 0.3 mmol; and the anti-cancer agent is irinotecan which is administered to the patient at from 40% to 60% of 180 mg/m², such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60% of 180 mg/m².

In one embodiment, the first predefined dosage of the UGT1A1 inhibitor is 0.3 mmol; and the first predefined dosage of the anti-cancer agent is from 40% to 60% of 180 mg/m², such as 50% of 180 mg/m².

In one embodiment, the second predefined dosage of the anti-cancer agent is from 40% to 60% of the recommended dose according to the Summary of Product Characteristics, such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60%.

In one embodiment, the second predefined dosage of the anti-cancer agent is from 40% to 60% of 180 mg/m², such as 50% of 180 mg/m².

In one embodiment, the second predefined dosage of the UGT1A1 inhibitor is 0.2 mmol; and the second predefined dosage of the anti-cancer agent is from 40% to 60% of 180 mg/m², such as 50% of 180 mg/m².

In one embodiment, the method further comprises administering a first predefined dosage of the UGT1A1 inhibitor to the patient having a bilirubin index below the threshold value.

In one embodiment, the method further comprises administering a second predefined dosage of the UGT1A1 inhibitor to the patient having a bilirubin index above the threshold value.

### Prediction methods

As demonstrated by Example 6, administering SCO-101 prior to initiating cancer therapy, and measuring the rate of unconjugated bilirubin normalization i.e. the bilirubin index, can be used to identify which patients are at increased risk of developing grade 3 or 4 neutropenia from cancer therapy, in particular from treatments comprising an UGT1A1 substrate, such as irinotecan and an UGT1A1 inhibitor, such as SCO-101.

The present disclosure further provides a method for predicting if a patient is at risk of developing grade 3 or 4 neutropenia in response to an anti-cancer agent, wherein the anti-cancer agent is an UGT1A1 substrate, said method comprising the consecutive steps of:
1) administering a first predefined dosage of an UGT1A1 inhibitor to the patient one or more times;
2) determining a first concentration of unconjugated bilirubin at a first time point;
3) discontinuing administration of the UGT1A1 inhibitor;
4) determining a second concentration of unconjugated bilirubin at a second time point which is subsequent in time to the first time point; and
wherein the patient is at risk of developing grade 3 or 4 neutropenia if the bilirubin index being the second concentration divided by the first concentration is above a threshold value; and/or
wherein the patient is not at risk of developing grade 3 or 4 neutropenia if the bilirubin index being the second concentration divided by the first concentration is below the threshold value.

In addition, the present disclosure provides a method for predicting if a patient has or is likely to have a RAS-like mutated cancer, wherein the anti-cancer agent is an UGT1A1 substrate, said method comprising the consecutive steps of:
1) administering a first predefined dosage of an UGT1A1 inhibitor to the patient one or more times; and
2) determining a first concentration of unconjugated bilirubin at a first time point;
3) discontinuing treatment with the UGT1A1 inhibitor;
4) determining a second concentration of unconjugated bilirubin at a second time point which is subsequent in time to the first time point; and
wherein the patient has or is likely to have a RAS-like mutated cancer if the bilirubin index being the second concentration divided by the first concentration is above a threshold value; and/or
wherein the patient does not have or is not likely to have a RAS-like mutated cancer if the bilirubin index being the second concentration divided by the first concentration is below the threshold value.

### Combination drug

The present disclosure provides in one embodiment, a combination drug comprising,
a) separately or together, 0.3 mmol of an UGT1A1 inhibitor, and 50% of 180 mg/m² of an anti-cancer agent which is an UGT1A1 substrate; or
b) separately or together, 0.2 mmol of an UGT1A1 inhibitor, and 50% of 180 mg/m² of an anti-cancer agent which is an UGT1A1 substrate.

In one embodiment, a combination drug is provided comprising,
a) separately or together, a first predefined dosage of an UGT1A1 inhibitor, and less than 180 mg/m² of an anti-cancer agent which is an UGT1A1 substrate; or
b) separately or together, a second predefined dosage of an UGT1A1 inhibitor, and less than 180 mg/m² of an anti-cancer agent which is an UGT1A1 substrate; wherein
the first predefined dosage of the UGT1A1 inhibitor is higher than the second predefined dosage of the UGT1A1 inhibitor.

In one embodiment, a combination drug is provided comprising, separately or together, an UGT1A1 inhibitor and an anti-cancer agent for use in the treatment of cancer in a patient; wherein the cancer is not a RAS mutated cancer; wherein the anti-cancer agent is an UGT1A1 substrate; and wherein the UGT1A1 inhibitor is SCO-101: 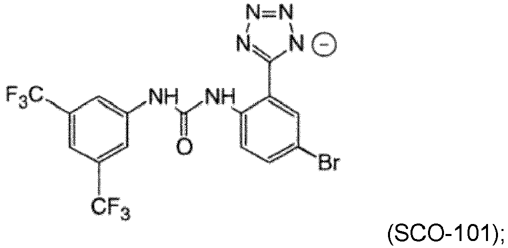 or a pharmaceutically acceptable salt thereof.

In one embodiment, a combination drug is provided comprising, separately or together, an UGT1A1 inhibitor and an anti-cancer agent for use in the treatment of cancer in a patient; wherein the cancer is a RAS wild-type; wherein the anti-cancer agent is an UGT1A1 substrate; and wherein the UGT1A1 inhibitor is SCO-101: 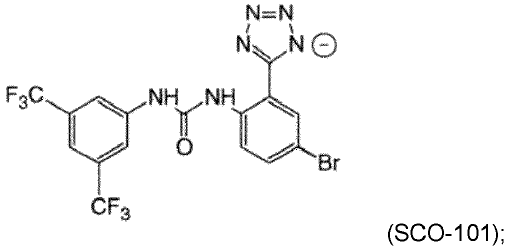 or a pharmaceutically acceptable salt thereof.

In one embodiment, a combination drug is provided comprising, separately or together, an UGT1A1 inhibitor and an anti-cancer agent for use in the treatment of cancer in a patient; wherein the cancer is a RAS mutated cancer; wherein the anti-cancer agent is an UGT1A1 substrate; and wherein the UGT1A1 inhibitor is SCO-101: 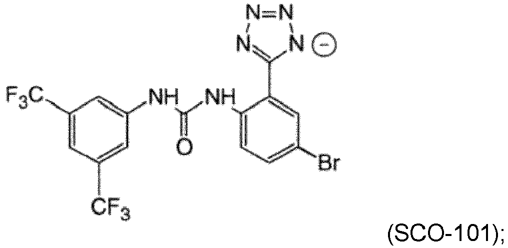 or a pharmaceutically acceptable salt thereof.

In one embodiment, a combination drug is provided comprising,
a) separately or together, 0.3 mmol of an UGT1A1 inhibitor, and 50% of 180 mg/m² of an anti-cancer agent which is an UGT1A1 substrate; or
b) separately or together, 0.2 mmol of an UGT1A1 inhibitor, and 50% of 180 mg/m² of an anti-cancer agent which is an UGT1A1 substrate;
wherein the UGT1A1 inhibitor is SCO-101: 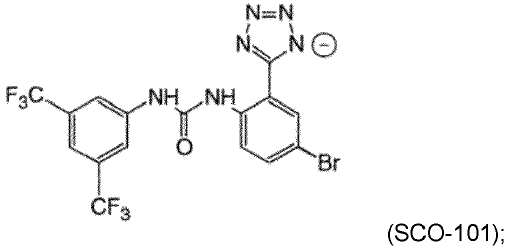 or a pharmaceutically acceptable salt thereof.

### Items I

1. A method of treating a patient having cancer comprising administering an UGT1A1 inhibitor and an anti-cancer agent to the patient; wherein the cancer is not a RAS mutated cancer.
2. A method of treating a patient having cancer comprising administering an UGT1A1 inhibitor and an anti-cancer agent to the patient; wherein the cancer is a RAS wild-type.
3. The method according to any one of the preceding items, wherein the cancer does not have a RAS mutation in a gene selected from the group consisting of: KRAS, HRAS, and NRAS.
4. The method according to any one of the preceding items, wherein the cancer does not have a KRAS mutation.
5. The method according to any one of the preceding items, wherein the UGT1A1 inhibitor is administered to the patient in a daily dosage of from 0.1 mmol to 0.4 mmol, such as from 0.1 mmol to 0.2 mmol, such as from 0.2 mmol to 0.3 mmol, such as from 0.3 mmol to 0.4 mmol, for example 0.2 mmol, or for example 0.3 mmol.
6. The method according to any one of the preceding items, wherein the UGT1A1 inhibitor is administered to the patient in a daily dosage of 0.3 mmol.
7. The method according to any one of the preceding items, wherein the UGT1A1 inhibitor is administered to the patient in a daily dosage of from 0.2 mmol to 0.4 mmol, for example 0.3 mmol; and the anti-cancer agent is administered to the patient at from 40% to 60% of the recommended dose according to the Summary of Product Characteristics, such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60%.
8. The method according to any one of the preceding items, wherein the UGT1A1 inhibitor is administered to the patient in a daily dosage of from 0.2 mmol to 0.4 mmol, for example 0.3 mmol; and the anti-cancer agent is irinotecan which is administered to the patient at from 40% to 60% of 180 mg/m², such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60% of 180 mg/m².
9. A method of treating a patient having cancer comprising administering an UGT1A1 inhibitor and an anti-cancer agent to the patient; wherein the cancer is a RAS mutated cancer.
10. The method according to item 9, wherein the cancer has a RAS mutation in a gene selected from the group consisting of: KRAS, HRAS, and NRAS.
11. The method according to any one of items 9-10, wherein the cancer has a KRAS mutation.
12. The method according to any one of the preceding items, wherein the UGT1A1 inhibitor is administered to the patient in a daily dosage of from 0.1 mmol to 0.4 mmol, such as from 0.1 mmol to 0.2 mmol, such as from 0.2 mmol to 0.3 mmol, such as from 0.3 mmol to 0.4 mmol, for example 0.2 mmol, or for example 0.3 mmol.
13. The method according to any one of the preceding items, wherein the UGT1A1 inhibitor is administered to the patient in a daily dosage of 0.2 mmol.
14. The method according to any one of the preceding items, wherein the UGT1A1 inhibitor is SCO-101: 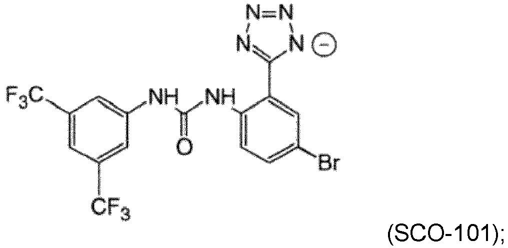 or a pharmaceutically acceptable salt thereof.
15. The method according to any one of the preceding items, wherein the UGT1A1 inhibitor is administered to the patient daily.
16. The method according to any one of the preceding items, wherein the anti-cancer agent is an UGT1A1 substrate.
17. The method according to any one of the preceding items, wherein the anti-cancer agent is a topoisomerase inhibitor.
18. The method according to any one of the preceding items, wherein the anti-cancer agent is a topoisomerase I inhibitor or topoisomerase II inhibitor.
19. The method according to any one of the preceding items, wherein the anti-cancer agent is a topoisomerase I inhibitor selected from the group consisting of: irinotecan, its active metabolite SN-38, and topotecan.
20. The method according to any one of the preceding items, wherein the anti-cancer agent is administered in combination with a further anti-cancer agent.
21. The method according to any one of the preceding items, wherein the anti-cancer agent is administered in combination with a further anti-cancer agent which is 5-fluorouracil.
22. The method according to any one of the preceding items, wherein the anti-cancer agent is administered in combination with 5-fluorouracil and folinic acid.
23. The method according to any one of the preceding items, wherein the anti-cancer agent is irinotecan and administered in combination with 5-fluorouracil and folinic acid.
24. The method according to any of the preceding items, wherein the cancer is a solid tumour, such as a solid tumour selected from sarcoma, carcinoma and lymphoma.
25. The method according to any of the preceding items, wherein the cancer is selected from the group consisting of Colorectal cancer, Breast cancer, lung cancer (non small cell lung cancer and small cell lung cancer), Glioblastomas, Head and neck cancers, Malignant melanomas, Basal cell skin cancer, Squamous cell skin cancer, Liver cancer, Pancreatic cancer, Prostate cancer, anal cancer, Cervix uteri cancer, Bladder cancer, Corpus uteri cancer, Ovarian cancer, Gall bladder cancer, Sarcomas, Leukemia's (myeloid and lymphatic), Lymphomas, Myelomatosis, and cholangiocarcinoma.
26. The method according to any of the preceding items, wherein the cancer is metastatic cancer.
27. The method according to any of the preceding items, wherein the cancer is colorectal cancer, such as metastatic colorectal cancer.
28. The method according to any one of the preceding items, wherein the cancer is pancreatic cancer, such as metastatic pancreatic cancer.
29. The method according to any of the preceding items, wherein the cancer is breast cancer, such as metastatic breast cancer.
30. The method according to any of the preceding items, wherein the cancer is a resistant cancer which is resistant to the anti-cancer agent when administered alone.
31. The method according to item 30, wherein the resistance is *de novo* resistance.
32. The method according to item 30, wherein resistance is acquired resistance.
33. The method according to any of items 30 to 32, wherein treatment with the UGT1A1 inhibitor re-sensitises the cancer to the anti-cancer agent.
34. The method according to any one of the preceding items, comprising a treatment cycle wherein the UGT1A1 inhibitor is administered to the patient daily from a first day to a sixth day, and the anti-cancer agent is administered to the patient from a fifth day to a seventh day.
35. The method according to any one of the preceding items, wherein the treatment cycle is repeated a number of times, such as 2 times or more, such as 3 times or more, such as 4 times or more, such as 5 times or more, such as 6 times or more, such as 7 times or more, such as 8 times or more, such as 9 times or more, such as 10 times or more, such as 11 times or more, such as 12 times or more, such as 13 times or more, such as 14 times or more, such as 15 times or more, such as 16 times or more, such as 17 times or more, such as 18 times or more, such as 19 times or more, such as 20 times or more, such as 21 times or more, such as 22 times or more, such as 23 times or more, such as 24 times or more, such as 25 times or more, such as 26 times or more, such as 27 times or more, such as 28 times or more, such as 29 times or more, such as 30 times or more, 31 times or more, such as 32 times or more, such as 33 times or more, such as 34 times or more, such as 35 times or more, such as 36 times or more, such as 37 times or more, such as 38 times or more, such as 39 times or more, such as 40 times or more, such as 41 times or more, such as 42 times or more, such as 43 times or more, such as 44 times or more, such as 45 times or more, such as 46 times or more, such as 47 times or more, such as 48 times or more, such as 49 times or more, such as 50 times or more, such as 51 times or more.
36. The method according to any one of the preceding items, further comprising administering to the patient a Granulocyte colony-stimulating factor (G-CSF).
37. The method according to any one of the preceding items, further comprising administering to the patient a Granulocyte colony-stimulating factor (G-CSF) prior to or simultaneously with administration of the anti-cancer agent.
38. The method according to any one of the preceding items, further comprising administering to the patient a Granulocyte colony-stimulating factor (G-CSF) prior to or simultaneously with administration of the anti-cancer agent and subsequent to administration of the UGT1A1 inhibitor within a treatment cycle, optionally wherein G-CSF is administered one or more times during the treatment cycle.
39. A method for identifying a treatment regimen for a patient having cancer with an UGT1A1 inhibitor and an anti-cancer agent; said method comprising determining if the patient has a RAS mutated cancer; and
   wherein if the cancer is not a RAS mutated cancer, the dosage regimen is a first predefined dosage of the UGT1A1 inhibitor and a first predefined dosage of the anti-cancer agent; or
   wherein if the cancer is a RAS mutated cancer, the dosage regimen is a second predefined dosage of the UGT1A1 inhibitor and a second predefined dosage of the anti-cancer agent;
   wherein the first and second predefined dosages of the anti-cancer agent are different; and/or
   wherein the first and second predefined dosages of the UGT1A1 inhibitor are different.
40. A method for identifying a treatment regimen for a patient having cancer and treating the patient with an UGT1A1 inhibitor and an anti-cancer agent; said method comprising determining if the patient has a RAS mutated cancer; and administering a first predefined dosage of the UGT1A1 inhibitor and a first predefined dosage of the anti-cancer agent to the patient that does not have a RAS mutated cancer; or
   administering a second predefined dosage of the UGT1A1 inhibitor and a second predefined dosage of the anti-cancer agent to the patient having a RAS mutated cancer;
   wherein the first and second predefined dosages of the anti-cancer agent are different; and/or
   wherein the first and second predefined dosages of the UGT1A1 inhibitor are different.
41. A method for selecting an anti-cancer agent dosage regimen for a cancer patient, wherein the anti-cancer agent is an UGT1A1 substrate; said method comprising the consecutive steps of:
   1) administering a first predefined dosage of an UGT1A1 inhibitor to the patient one or more times;
   2) determining the plasma concentration of unconjugated bilirubin in the patient at a first time point;
   3) discontinuing administration of the UGT1A1 inhibitor;
   4) determining the plasma concentration of unconjugated bilirubin at a second time point which is subsequent in time to the first time point;
   5) generating a bilirubin index being the second plasma concentration of unconjugated bilirubin divided by the first plasma concentration of unconjugated bilirubin, and

   wherein if the bilirubin index is below a threshold value, selecting a first predefined dosage of the anti-cancer agent as dosage regimen; or
   wherein if the bilirubin index is above a threshold value, selecting a second predefined dosage of the anti-cancer agent as dosage regimen;
   wherein the first and second predefined dosages of the anti-cancer agent are different.
42. A method for identifying an anti-cancer agent dosage regimen for a cancer patient and treating the cancer patient, wherein the anti-cancer agent is an UGT1A1 substrate; said method comprising the consecutive steps of:
   1) administering a first predefined dosage of an UGT1A1 inhibitor to the patient one or more times;
   2) determining the plasma concentration of unconjugated bilirubin in the patient at a first time point;
   3) discontinuing administration of the UGT1A1 inhibitor;
   4) determining the plasma concentration of unconjugated bilirubin at a second time point which is subsequent in time to the first time point;
   5) generating a bilirubin index being the second plasma concentration of unconjugated bilirubin divided by the first plasma concentration of unconjugated bilirubin, and
   6) administering a first predefined dosage of the anti-cancer agent to the patient having a bilirubin index below a threshold value; or
   7) administering a second predefined dosage of the anti-cancer agent to the patient having a bilirubin index above the threshold value;
   wherein the first and second predefined dosages of the anti-cancer agent are different.
43. The method according to any one of items 39-42, wherein the second predefined dosage of the anti-cancer agent is lower than the first predefined dosage of the anti-cancer agent.
44. The method according to any one of items 39-42, wherein the second predefined dosage of the UGT1A1 inhibitor is lower than the first predefined dosage of the UGT1A1 inhibitor.
45. The method according to any one of items 39-44, wherein the first predefined dosage of the UGT1A1 inhibitor is from 0.1 mmol to 0.4 mmol, such as from 0.1 mmol to 0.2 mmol, such as from 0.2 mmol til 0.3 mmol, such as from 0.3 mmol to 0.4 mmol.
46. The method according to any one of items 39-45, wherein the first predefined dosage of the anti-cancer agent is from 40% to 60% of the recommended dose according to the Summary of Product Characteristics, such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60%.
47. The method according to any one of items 39-46, wherein the first predefined dosage of the anti-cancer agent is from 40% to 60% of 180 mg/m², such as 50% of 180 mg/m².
48. The method according to any one of items 39-47, wherein the first predefined dosage of the UGT1A1 inhibitor is from 0.1 mmol to 0.4 mmol, such as from 0.1 mmol to 0.2 mmol, such as from 0.2 mmol til 0.3 mmol, such as from 0.3 mmol to 0.4 mmol.
49. The method according to any one of items 39-48, wherein the first predefined dosage of the UGT1A1 inhibitor is 0.3 mmol; and the first predefined dosage of the anti-cancer agent is from 40% to 60% of 180 mg/m², such as 50% of 180 mg/m².
50. The method according to any one of items 39-49, wherein the second predefined dosage of the anti-cancer agent is from 40% to 60% of the recommended dose according to the Summary of Product Characteristics, such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60%.
51. The method according to any one of items 39-50, wherein the second predefined dosage of the anti-cancer agent is from 40% to 60% of 180 mg/m², such as 50% of 180 mg/m².
52. The method according to any one of items 39-51, wherein the second predefined dosage of the UGT1A1 inhibitor is 0.2 mmol; and the second predefined dosage of the anti-cancer agent is from 40% to 60% of 180 mg/m², such as 50% of 180 mg/m².
53. The method according to any one of items 41-52, wherein no UGT1A1 inhibitor is administered to the patient between steps 2) and 4).
54. The method according to any one of items 41-53, wherein the second time point is 5 days subsequent to the first time point.
55. The method according to any one of items 41-54, wherein the threshold value is from 0.3 to 0.5, such as from 0.3 to 0.4, such as from 0.4 to 0.5, for example 0.4.
56. The method according to any one of items 41-55, further comprising administering a first predefined dosage of the UGT1A1 inhibitor to the patient having a bilirubin index below the threshold value.
57. The method according to any one of items 41-56, further comprising administering a second predefined dosage of the UGT1A1 inhibitor to the patient having a bilirubin index above the threshold value.
58. A method for predicting if a patient is at risk of developing grade 3 or 4 neutropenia in response to an anti-cancer agent, wherein the anti-cancer agent is an UGT1A1 substrate, said method comprising the consecutive steps of:
   1) administering a first predefined dosage of an UGT1A1 inhibitor to the patient one or more times;
   2) determining a first concentration of unconjugated bilirubin at a first time point;
   3) discontinuing administration of the UGT1A1 inhibitor;
   4) determining a second concentration of unconjugated bilirubin at a second time point which is subsequent in time to the first time point; and
   wherein the patient is at risk of developing grade 3 or 4 neutropenia if the bilirubin index being the second concentration divided by the first concentration is above a threshold value; and/or
   wherein the patient is not at risk of developing grade 3 or 4 neutropenia if the bilirubin index being the second concentration divided by the first concentration is below the threshold value.
59. A method for predicting if a patient has or is likely to have a RAS-like mutated cancer, wherein the anti-cancer agent is an UGT1A1 substrate, said method comprising the consecutive steps of:
   1) administering a first predefined dosage of an UGT1A1 inhibitor to the patient one or more times; and
   2) determining a first concentration of unconjugated bilirubin at a first time point;
   3) discontinuing treatment with the UGT1A1 inhibitor;
   4) determining a second concentration of unconjugated bilirubin at a second time point which is subsequent in time to the first time point; and
   wherein the patient has or is likely to have a RAS-like mutated cancer if the bilirubin index being the second concentration divided by the first concentration is above a threshold value; and/or
   wherein the patient does not have or is not likely to have a RAS-like mutated cancer if the bilirubin index being the second concentration divided by the first concentration is below the threshold value.
60. A combination drug comprising,
   a) separately or together, 0.3 mmol of an UGT1A1 inhibitor, and 50% of 180 mg/m² of an anti-cancer agent which is an UGT1A1 substrate; or
   b) separately or together, 0.2 mmol of an UGT1A1 inhibitor, and 50% of 180 mg/m² of an anti-cancer agent which is an UGT1A1 substrate.

### Items II

1. A method of treating a patient having cancer comprising administering a first predefined dosage of an UGT1A1 inhibitor and a first predefined dosage of an anti-cancer agent to the patient; wherein the cancer is not a RAS mutated cancer.
2. A method of treating a patient having cancer comprising administering a first predefined dosage of an UGT1A1 inhibitor and a first predefined dosage of an anti-cancer agent to the patient; wherein the cancer is a RAS wild-type.
3. The method according to any one of the preceding items, wherein the cancer does not have a RAS mutation in a gene selected from the group consisting of: KRAS, HRAS, and NRAS.
4. The method according to any one of the preceding items, wherein the cancer does not have a KRAS mutation.
5. The method according to any one of the preceding items, wherein the first predefined dosage of the UGT1A1 inhibitor is higher compared to a second predefined dosage of the UGT1A1 inhibitor administered to a patient having a cancer which is RAS mutated.
6. The method according to any one of the preceding items, wherein the first predefined dosage of the anti-cancer agent is higher compared to a second predefined dosage of the anti-cancer agent administered to a patient having a cancer which is RAS mutated.
7. The method according to any one of the preceding items, wherein the first predefined dosage of the UGT1A1 inhibitor is 0.30 mmol.
8. The method according to any one of the preceding items, wherein the first predefined dosage of the anti-cancer agent is below 180 mg/m² irinotecan or an equimolar dose of any other anti-cancer agent.
9. The method according to any one of the preceding items, wherein the first predefined dosage of the UGT1A1 inhibitor is 0.30 mmol, the anti-cancer agent is irinotecan; and the first predefined dosage of irinotecan is below 180 mg/m².
10. A method of treating a patient having cancer comprising administering a second predefined dosage of an UGT1A1 inhibitor and a second predefined dosage of an anti-cancer agent to the patient; wherein the cancer is a RAS mutated cancer.
11. The method according to item 10, wherein the cancer has a RAS mutation in a gene selected from the group consisting of: KRAS, HRAS, and NRAS.
12. The method according to any one of items 10-11, wherein the cancer has a KRAS mutation.
13. The method according to any one of items 10-12, wherein the second predefined dosage of the UGT1A1 inhibitor is lower compared to the first predefined dosage of the UGT1A1 inhibitor administered to a patient having a cancer which is not a RAS mutated cancer.
14. The method according to any one of items 10-13, wherein the second predefined dosage of the anti-cancer agent is lower compared to the first predefined dosage of the anti-cancer agent administered to a patient having a cancer which is not a RAS mutated cancer.
15. The method according to any one of the preceding items, wherein the second predefined dosage of the UGT1A1 inhibitor is below 0.30 mmol.
16. The method according to any one of the preceding items, wherein the second predefined dosage of the anti-cancer agent is below 180 mg/m² irinotecan or an equimolar dose of any other anti-cancer agent.
17. The method according to any one of the preceding items, wherein the second predefined dosage of the UGT1A1 inhibitor is below 0.30 mmol, the anti-cancer agent is irinotecan; and the second predefined dosage of irinotecan is below 180 mg/m².
18. The method according to any one of the preceding items, wherein the anti-cancer agent is administered to the patient at a dosage which is lower than the recommended dose according to the Summary of Product Characteristics.
19. The method according to any one of the preceding items, wherein the anti-cancer agent is administered to the patient at a dosage which is lower than the previously well tolerated (PWT) dosage of the anti-cancer agent when administered to the patient without an UGT1A1 inhibitor.
20. The method according to any one of the preceding items, wherein the anti-cancer agent is irinotecan which is administered to the patient at a dosage which is lower than 180 mg/m².
21. The method according to any one of the preceding items, wherein the UGT1A1 inhibitor is SCO-101: 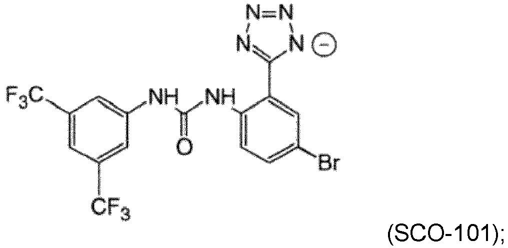 or a pharmaceutically acceptable salt thereof.
22. The method according to any one of the preceding items, wherein the UGT1A1 inhibitor is administered to the patient daily.
23. The method according to any one of the preceding items, wherein the anti-cancer agent is an UGT1A1 substrate.
24. The method according to any of the preceding items, wherein the anti-cancer agent is selected from the group consisting of: a topoisomerase inhibitor, an anti-hormone agent, an alkylating agent, a mitotic inhibitor, an antimetabolite, an anti-tumour antibiotic, a corticosteroid, a targeted anti-cancer therapy, a differentiating agent, and an immunotherapy.
25. The method according to any one of the preceding items, wherein the anti-cancer agent is a topoisomerase inhibitor.
26. The method according to any one of the preceding items, wherein the anti-cancer agent is a topoisomerase I inhibitor or topoisomerase II inhibitor.
27. The method according to any one of the preceding items, wherein the anti-cancer agent is a topoisomerase I inhibitor selected from the group consisting of:
   irinotecan, its active metabolite SN-38, and topotecan.
28. The method according to any of the preceding items wherein the anti-cancer agent is an anti-hormone agent.
29. The method according to any one of the preceding items, wherein the anti-cancer agent is an anti-estrogen, such as fulvestrant or tamoxifen.
30. The method according to any of the preceding items wherein the anti-cancer agent is an alkylating agent, such as temozolomide.
31. The method according to any of the preceding items wherein the anti-cancer agent is a mitotic inhibitor, such as paclitaxel or docetaxel.
32. The method according to any of the preceding items wherein the anti-cancer agent is an antimetabolite, such as 5-fluorouracil (5-FU).
33. The method according to any one of the preceding items, wherein the anti-cancer agent is administered in combination with a further anti-cancer agent.
34. The method according to any one of the preceding items, wherein the anti-cancer agent is administered in combination with a further anti-cancer agent which is 5-fluorouracil.
35. The method according to any one of the preceding items, wherein the anti-cancer agent is administered in combination with 5-fluorouracil and folinic acid.
36. The method according to any one of the preceding items, wherein the anti-cancer agent is irinotecan and administered in combination with 5-fluorouracil and folinic acid.
37. The method according to any of the preceding items, wherein the cancer is a solid tumour, such as a solid tumour selected from sarcoma, carcinoma and lymphoma.
38. The method according to any of the preceding items, wherein the cancer is selected from the group consisting of Colorectal cancer, Breast cancer, lung cancer (non small cell lung cancer and small cell lung cancer), Glioblastomas, Head and neck cancers, Malignant melanomas, Basal cell skin cancer, Squamous cell skin cancer, Liver cancer, Pancreatic cancer, Prostate cancer, anal cancer, Cervix uteri cancer, Bladder cancer, Corpus uteri cancer, Ovarian cancer, Gall bladder cancer, Sarcomas, Leukemia's (myeloid and lymphatic), Lymphomas, Myelomatosis, and cholangiocarcinoma.
39. The method according to any of the preceding items, wherein the cancer is metastatic cancer.
40. The method according to any of the preceding items, wherein the cancer is colorectal cancer, such as metastatic colorectal cancer.
41. The method according to any one of the preceding items, wherein the cancer is pancreatic cancer, such as metastatic pancreatic cancer.
42. The method according to any of the preceding items, wherein the cancer is breast cancer, such as metastatic breast cancer.
43. The method according to any of the preceding items, wherein the cancer is a resistant cancer which is resistant to the anti-cancer agent.
44. The method according to item 43, wherein resistance is *de novo* resistance.
45. The method according to item 43, wherein resistance is acquired resistance.
46. The method according to any of items 43-45, wherein treatment with the UGT1A1 inhibitor re-sensitises the cancer to the anti-cancer agent.
47. The method according to any one of the preceding items, comprising a treatment cycle wherein the UGT1A1 inhibitor is administered to the patient daily from a first day to a sixth day, and the anti-cancer agent is administered to the patient from a fifth day to a seventh day.
48. The method according to any one of the preceding items, wherein the treatment cycle is repeated a number of times, such as 2 times or more, such as 3 times or more, such as 4 times or more, such as 5 times or more, such as 6 times or more, such as 7 times or more, such as 8 times or more, such as 9 times or more, such as 10 times or more, such as 11 times or more, such as 12 times or more, such as 13 times or more, such as 14 times or more, such as 15 times or more, such as 16 times or more, such as 17 times or more, such as 18 times or more, such as 19 times or more, such as 20 times or more, such as 21 times or more, such as 22 times or more, such as 23 times or more, such as 24 times or more, such as 25 times or more, such as 26 times or more, such as 27 times or more, such as 28 times or more, such as 29 times or more, such as 30 times or more, 31 times or more, such as 32 times or more, such as 33 times or more, such as 34 times or more, such as 35 times or more, such as 36 times or more, such as 37 times or more, such as 38 times or more, such as 39 times or more, such as 40 times or more, such as 41 times or more, such as 42 times or more, such as 43 times or more, such as 44 times or more, such as 45 times or more, such as 46 times or more, such as 47 times or more, such as 48 times or more, such as 49 times or more, such as 50 times or more, such as 51 times or more.
49. The method according to any one of the preceding items, further comprising administering to the patient a Granulocyte colony-stimulating factor (G-CSF).
50. The method according to any one of the preceding items, further comprising administering to the patient a Granulocyte colony-stimulating factor (G-CSF) prior to or simultaneously with administration of the anti-cancer agent.
51. The method according to any one of the preceding items, further comprising administering to the patient a Granulocyte colony-stimulating factor (G-CSF) prior to or simultaneously with administration of the anti-cancer agent and subsequent to administration of the UGT1A1 inhibitor within a treatment cycle, optionally wherein G-CSF is administered one or more times during the treatment cycle.
52. A method for identifying a treatment regimen for a patient having cancer with an UGT1A1 inhibitor and an anti-cancer agent; said method comprising determining if the patient has a RAS mutated cancer; and
   wherein if the cancer is not a RAS mutated cancer, the dosage regimen is a first predefined dosage of the UGT1A1 inhibitor and a first predefined dosage of the anti-cancer agent; or
   wherein if the cancer is a RAS mutated cancer, the dosage regimen is a second predefined dosage of the UGT1A1 inhibitor and a second predefined dosage of the anti-cancer agent;
   wherein the first and second predefined dosages of the anti-cancer agent are different; and/or
   wherein the first and second predefined dosages of the UGT1A1 inhibitor are different.
53. A method for identifying a treatment regimen for a patient having cancer and treating the patient with an UGT1A1 inhibitor and an anti-cancer agent; said method comprising determining if the patient has a RAS mutated cancer; and administering a first predefined dosage of the UGT1A1 inhibitor and a first predefined dosage of the anti-cancer agent to the patient that does not have a RAS mutated cancer; or
   administering a second predefined dosage of the UGT1A1 inhibitor and a second predefined dosage of the anti-cancer agent to the patient having a RAS mutated cancer;
   wherein the first and second predefined dosages of the anti-cancer agent are different; and/or
   wherein the first and second predefined dosages of the UGT1A1 inhibitor are different.
54. The method according to any one of items 52-53, wherein the first and second predefined dosages of the anti-cancer agent are the same; and wherein the first and second predefined dosages of the UGT1A1 inhibitor are different.
55. The method according to any one of items 52-53, wherein the first and second predefined dosages of the anti-cancer agent are the same; and wherein the first predefined dosage of the UGT1A1 inhibitor is higher than the second predefined dosage of the UGT1A1 inhibitor.
56. The method according to any one of items 52-53, wherein the first and second predefined dosages of the UGT1A1 inhibitor are the same; and the first and second predefined dosages of the anti-cancer agent are different.
57. The method according to any one of items 52-53, wherein the first and second predefined dosages of the UGT1A1 inhibitor are the same; and wherein the first predefined dosage of the anti-cancer agent is higher than the second predefined dosage of the anti-cancer agent.
58. A method for selecting an anti-cancer agent dosage regimen for a cancer patient, wherein the anti-cancer agent is an UGT1A1 substrate; said method comprising the consecutive steps of:
   1) administering a first predefined dosage of an UGT1A1 inhibitor to the patient one or more times;
   2) determining the plasma concentration of unconjugated bilirubin in the patient at a first time point;
   3) discontinuing administration of the UGT1A1 inhibitor;
   4) determining the plasma concentration of unconjugated bilirubin at a second time point which is subsequent in time to the first time point;
   5) generating a bilirubin index being the second plasma concentration of unconjugated bilirubin divided by the first plasma concentration of unconjugated bilirubin, and

   wherein if the bilirubin index is below a threshold value, selecting a first predefined dosage of the anti-cancer agent as dosage regimen; or
   wherein if the bilirubin index is above a threshold value, selecting a second predefined dosage of the anti-cancer agent as dosage regimen;
   wherein the first and second predefined dosages of the anti-cancer agent are different.
59. A method for identifying an anti-cancer agent dosage regimen for a cancer patient and treating the cancer patient, wherein the anti-cancer agent is an UGT1A1 substrate; said method comprising the consecutive steps of:
   1) administering a first predefined dosage of an UGT1A1 inhibitor to the patient one or more times;
   2) determining the plasma concentration of unconjugated bilirubin in the patient at a first time point;
   3) discontinuing administration of the UGT1A1 inhibitor;
   4) determining the plasma concentration of unconjugated bilirubin at a second time point which is subsequent in time to the first time point;
   5) generating a bilirubin index being the second plasma concentration of unconjugated bilirubin divided by the first plasma concentration of unconjugated bilirubin, and
   6) administering a first predefined dosage of the anti-cancer agent to the patient having a bilirubin index below a threshold value; or
   7) administering a second predefined dosage of the anti-cancer agent to the patient having a bilirubin index above the threshold value;
   wherein the first and second predefined dosages of the anti-cancer agent are different.
60. The method according to any one of items 52-59, wherein the second predefined dosage of the anti-cancer agent is lower than the first predefined dosage of the anti-cancer agent.
61. The method according to any one of items 52-60, wherein the first predefined dosage of the anti-cancer agent is lower than 180 mg/m².
62. The method according to any one of items 52-61, wherein the second predefined dosage of the anti-cancer agent is lower than 180 mg/m².
63. The method according to any one of items 52-62, wherein the first and second predefined dosages of the anti-cancer agent are the same; and wherein the second predefined dosage of the UGT1A1 inhibitor is lower than the first predefined dosage of the UGT1A1 inhibitor.
64. The method according to any one of items 52-63, wherein the first predefined dosage of the UGT1A1 inhibitor is 0.30 mmol.
65. The method according to any one of items 58-63, wherein no UGT1A1 inhibitor is administered to the patient between steps 2) and 4).
66. The method according to any one of items 58-65, wherein the second time point is 5 days subsequent to the first time point.
67. The method according to any one of items 58-66, wherein the threshold value is from 0.3 to 0.5, such as from 0.3 to 0.4, such as from 0.4 to 0.5, for example 0.4.
68. The method according to any one of items 58-68, further comprising administering a first predefined dosage of the UGT1A1 inhibitor to the patient having a bilirubin index below the threshold value.
69. The method according to any one of items 58-68, further comprising administering a second predefined dosage of the UGT1A1 inhibitor to the patient having a bilirubin index above the threshold value.
70. A method for predicting if a patient is at risk of developing neutropenia, such as grade 3 or 4 neutropenia, in response to an anti-cancer agent, wherein the anti-cancer agent is an UGT1A1 substrate, said method comprising the consecutive steps of:
   1) administering a first predefined dosage of an UGT1A1 inhibitor to the patient one or more times;
   2) determining a first concentration of unconjugated bilirubin at a first time point;
   3) discontinuing administration of the UGT1A1 inhibitor;
   4) determining a second concentration of unconjugated bilirubin at a second time point which is subsequent in time to the first time point; and

   wherein the patient is at risk of developing grade 3 or 4 neutropenia if the bilirubin index being the second concentration divided by the first concentration is above a threshold value; and/or
   wherein the patient is not at risk of developing grade 3 or 4 neutropenia if the bilirubin index being the second concentration divided by the first concentration is below the threshold value.
71. A method for predicting if a patient has or is likely to have a RAS-like mutated cancer, wherein the anti-cancer agent is an UGT1A1 substrate, said method comprising the consecutive steps of:
   1) administering a first predefined dosage of an UGT1A1 inhibitor to the patient one or more times; and
   2) determining a first concentration of unconjugated bilirubin at a first time point;
   3) discontinuing treatment with the UGT1A1 inhibitor;
   4) determining a second concentration of unconjugated bilirubin at a second time point which is subsequent in time to the first time point; and
   wherein the patient has or is likely to have a RAS-like mutated cancer if the bilirubin index being the second concentration divided by the first concentration is above a threshold value; and/or
   wherein the patient does not have or is not likely to have a RAS-like mutated cancer if the bilirubin index being the second concentration divided by the first concentration is below the threshold value.
72. A combination drug comprising,
   a) separately or together, a first predefined dosage of an UGT1A1 inhibitor, and less than 180 mg/m² of an anti-cancer agent which is an UGT1A1 substrate; or
   b) separately or together, a second predefined dosage of an UGT1A1 inhibitor, and less than 180 mg/m² of an anti-cancer agent which is an UGT1A1 substrate;
   wherein the first predefined dosage of the UGT1A1 inhibitor is higher than the second predefined dosage of the UGT1A1 inhibitor.

### Examples

### Example 1: Clinical study of the combination of SCO-101 with FOLFIRI in cancer patients

### Materials & Methods

We performed a clinical study investigating drug candidate SCO-101 in combination with approved chemotherapy regimen FOLFIRI (combination of Irinotecan, 5-flourouracil (5-FU) and leucovorin (folinic acid)) for treatment of metastatic colorectal cancer in patients who's metastatic cancer had developed resistance to FOLFIRI. Patients had previously been treated with FOLFIRI and experienced benefit of the treatment, but had since progressed, i.e. the cancer cells had acquired resistance to FOLFIRI chemotherapy treatment.

Patients were treated in 14 day cycles. In each treatment cycle, a daily dose of SCO-101 was administered for 6 consecutive days on days 1 to 6. SCO-101 was administered as oral tablets. In cohort 1, patients received 150 mg SCO-101 in cohort 2, patients received 100 mg SCO-101. FOLFIRI was administered on day 5 to 7 according to recommended dosage described the local Summary of Product Characteristics. The recommended doses are 180 mg/m² for Irinotecan, 200 mg/m² for leucovorin and 400 mg/m² as a bolus dose followed by 2.400 mg/m² as a 46-hour infusion The dose of FOLFIRI was calculated according to the weight of the patient and adjusted to the patients tolerability in previous treatments, i.e. the dosage of FOLFIRI had already been reduced from the recommended dose level in most patients. The patient specific dose levels can be seen in Fig. 2. Irinotecan was administered as a 90-minutes intravenous infusion on day 5. Leucovorin was administered as a 2-hour intravenous infusion on day 5 concurrently with Irinotecan. Immediately following completion of Leucovorin administration, a bolus intravenous injection of 5-FU was administered followed by a 46-hour infusion (via pump) on days 5, 6 and 7 which concluded the treatment. The treatment cycle was repeated every 14 days. Treatment effect was evaluated after every 4 treatment cycles using CT- or MRI-scan.

Due to repeated observations of neutropenia, prophylactic treatment with Granulocyte colony-stimulating factor (G-CSF) was implemented for all patients on day 7 in each treatment cycle.

The following blood samples were collected for analysis of selected biochemical parameters:
Bilirubin: Blood sampling for analysis of bilirubin (total, unconjugated and conjugated) were withdrawn at Screening (up to 4 weeks prior start of treatment), day 5, day 7 and day 12 in the first treatment cycle.
Pharmacokinetics of SCO-101 and Irinotecan: Blood sampling for pharmacokinetics were withdrawn according to below schedule:
   Day 1 (pre-SCO-101 administration, 1; 4; 5; 6; 8)
   Day 2 (24 hours (pre-SCO-101 administration))
   Day 5 (pre-SCO-101 administration, 1; 4; 5; 6; 8)
   Day 6 (24 hours (pre-SCO-101 administration)),
   Day 7 (48 hours),
   Day 9 (96 hours).
Biochemical safety assessment of hematological and chemistry parameters for safety assessment: Blood sampling for safety assessment of biochemical parameters were withdrawn at Screening (up to 4 weeks prior to start of treatment, day 1, day 5 and day 12 in cycle 1.
   - Hematology: Hemoglobin, Hematocrit, MCV, MCHC, MCH, RBC count, WBC count, WBC Differential, Absolute lymphocyte count, Absolute neutrophil count and Platelet count
   - Coagulation: PT, and INR
   - Chemistry: Alkaline Phosphatase, ALT, AST (not mandatory), Gamma-glutamyl transferase (GGT), Albumin, Total Protein, Creatinine, Urea (BUN), Uric acid, Total Creatinine Kinase (Ck) (if total Ck is above the ULN for the laboratory, Troponin-T or Troponin-I will be determined), Sodium, Potassium, Calcium, Chloride, Bicarbonate, Glucose, Triglycerides (TG), Amylase and CEA.

Cancer RAS mutation status was collected from patient journals and was based on assessment of previous biopsies from patients standard treatment course.

Cohort 1 was initiated with a dose of 150 mg SCO-101 in combination with FOLFIRI at a dose of 80% of the FOLFIRI dose the patients had previously tolerated well (PWT). The first two patients tolerated this dose well, but developed neutropenia in cycle two, where the dose of FOLFIRI had been increased to 100%. The third patient developed neutropenia after cycle 1. The Alliance Data and Safety Monitoring Board (DSMB) convened after treatment of the first 3 patients and decided that all patients should be treated with a dose of 80% FOLFIRI of PWT

The fourth patient received treatment with 80% FOLFIRI PWT and developed neutropenia after cycle 1. The DSMB convened and decided that additional 3 patients should be included and treated with 65% PWT FOLFIRI dose.

The fifth patient did not experience neutropenia during cycle 1, but developed short term neutropenia grade 2 in cycle 2 resulting in postponement of study treatment with one week. The sixth patient developed grade 4 neutropenia and the 7th patient did not experience any grade 3 or grade 4 adverse reactions. The DSMB convened and decided to implement prophylactic use of g-CSF and to include additional 2 patients to be treated at 65% PWT FOLFIRI (patient 7 had received prophylactic g-CSF) constituting a sub-cohort with 3 patients receiving 65% PWT FOLFIRI + prophylactic g-CSF.

The eight patient developed grade 4 neutropenia that was resolved on day 1 in cycle 2, however the patient was at this point hospitalized due to additional adverse reactions (back pain and staphylococcus epidermis infection) and treatment for cycle 2 was postponed. The events observed for this patient were considered a DLT (dose-limiting toxicity). The ninth patient did not experience grade 3 or grade 4 neutropenia. Hereafter the DSMB decided that additional 3 patients should be treated at the 65% PWT FOLFIRI dose level + prophylactic g-CSF constituting a sub-cohort with 3+3 patients treated with 65% PWT FOLFIRI + prophylactic g-CSF.

The tenth patient experienced grade 4 neutropenia which however had resolved before the beginning of cycle 2 (no treatment impact). The eleventh patient experienced grade 4 febrile neutropenia and was hospitalized resulting in postponement of treatment for cycle 2. Sponsor reviewed the patient data and found that the patient had received a dose corresponding to 89% PWT FOLFIRI (irinotecan and 5-FU dose) dosage, as the site had erroneously calculated the dose based only on the patients' body surface area (BSA) and not corrected it based on dose previously tolerated (PWT). The patient has resumed treatment in the study after the febrile neutropenia resolved and tolerated a dose of 65% PWT FOLFIRI well for treatment cycles 3 and 4. The first cycle of treatment after dose adjustment to 65% PWT FOLFIRI is used to evaluate dose-limiting toxicities (DLTs) for this patient. The twelfth patient initiated treatment with SCO-101 in cycle 1, however did not initiate chemotherapy treatment due to poor health and suspicion of COVID-19 infection (no treatment causality). The patient restarted treatment for cycle 2 and this cycle was used for assessment of DLTs. The patient developed grade 4 neutropenia and febrile neutropenia requiring prolonged hospitalization during cycle 2 resulting in treatment postponement. The patient did not resume treatment and has been withdrawn from the study. The events observed for this patient are considered a DLT.

With two DLTs in the last 6 patients, the DSMB concluded that a dose reduction of SCO-101 was required and cohort 2 was therefore initiated with 100 mg SCO-101 in combination with FOLFIRI. It was further identified that a dose of 65% PWT dose corresponded to approximately 50% of the recommended dose, and to reduce complexity in dose calculation of FOLFIRI, it was decided to continue with 50% of the recommended dose for subsequent patients.

After completion of cohort 1, data evaluation showed a very clear separation of patients by RAS status, i.e. patients with RAS mutation presented with bilirubin index above 0.4 (bilirubin serum concentration on day 12 divided with bilirubin serum concentration on day 7) in cycle 1 and did not tolerate combination treatment of SCO-101 and FOLFIRI well.

Six out of the twelve patients in cohort 1 presented with RAS mutation. Five out of six RAS mutated patients have developed grade 4 neutropenia after the first combination with SCO-101 and FOLFIRI. Only one patient did not develop grade 4 neutropenia. The RAS mutated patients have received an accumulated 11 out of 24 treatments during the first 4 treatment cycles. Of these 11 treatments, 6 resulted in grade 3 or grade 4 neutropenia and only 4 have been tolerated reasonably well - three of these were dosed to patient no. 5 while one was dosed to patient no 10. Dose reductions to 65% of the previously well tolerated dose level did not prevent dose limiting toxicity in the form of neutropenia.

Contrary, the six RAS wild type patients from cohort 1 have tolerated the combination of SCO-101 and FOLFIRI well. As described, a dose reduction is required for the subpopulation as well, but all patients tolerate combination of 150 mg SCO-101 and 65% PWT FOLFIRI dose without increased risk of severe neutropenia. A cumulative comparison of the number of treatment cycles completed (all treatment cycles where both SCO-101 and FOLFIRI has been administered) and the associated number of grade 3 or grade 4 neutropenia observed is included in Fig. 3.

Cohort 2 has currently included 4 patients treated with 100 mg SCO-101 and half the recommended dose of FOLFIRI. 2 patients are RAS wild type and 2 patients are RAS mutated. This dose level appears to be well tolerated, and no patients have experienced any grade 3 or grade 4 neutropenia or any other DLTs.

### Example 2: Treatment of patients having a cancer which is not RAS mutated

### Materials & Methods

This example is based on the clinical study of Example 1.

### Results

Administering SCO-101 and FOLFIRI over the course of 7 days as described in Example 1 was observed to be reasonably well tolerated by patients having a cancer without a RAS mutation in their primary tumour from initial diagnosis, after the dose of FOLFIRI had been adjusted to 65% of the previously well tolerated dose level. In cohort 1, 6 patients have completed a total of 43 out of 51 possible treatment cycles and patients which are RAS wild type show a significantly longer progression free treatment period compared to RAS mutated patients. In Fig. 1 a Kaplan-Meier plot of the patients from cohort 1 show a clear difference in progression free treatment period for patients with RAS wild type (i.e. without a RAS mutated cancer) compared to RAS mutated patients. The patient with the longest treatment time has been on treatment for more than 200 days. Dose reductions from the recommended (REC) and previously well tolerated (PWT) dose level are required as dosing between 80% to 100% PWT dose level resulted in severe neutropenia in some patients, however a dose level of 65% PWT appear well tolerated with few adverse reactions. A total of 4 cases of neutropenia was observed in treatment cycle 1 (Fig. 3) and 5 cases in total in all treatment cycles. In general the treatment combination of SCO-101 and FOLFIRI was observed to be well tolerated in RAS wild type patients. Patients having a RAS mutated cancer appear to require a different treatment regimen than the RAS wild type patients.

### Conclusion

The present example demonstrates that patients having a cancer which is not RAS mutated, tolerate the combination treatment of SCO-101 and FOLFIRI well and appear to have a significant benefit compared to patients having a RAS mutated cancer.

### Example 3: Treatment of patients having a RAS mutated cancer

### Materials & Methods

This example is based on the clinical study of Example 1.

### Results

Administering SCO-101 and FOLFIRI over the course of 7 days as described in Example 1 was observed to be poorly tolerated by patients with a cancer having a RAS mutation in their primary tumour from initial diagnosis. Six out of the twelve patients in cohort 1 (150 mg SCO-101) presented with RAS mutation. Five out of six RAS mutated patients have developed grade 4 neutropenia after the first combination with SCO-101 and FOLFIRI. Only one patient did not develop grade 4 neutropenia.

The patients with a RAS mutated cancer have received an accumulated 11 out of 24 treatments during the first 4 treatment cycles. Of these 11 treatments, 6 resulted in grade 3 or grade 4 neutropenia and only 4 have been tolerated reasonably well - three of these were dosed to patient no. 5 while one was dosed to patient no 10. Dose reductions to 65% of the previously well tolerated dose level did not prevent dose limiting toxicity in the form of neutropenia. Dose reductions to 65% PWT did not reduce the risk of neutropenia and reduction of the dose of SCO-101 to 100 mg was necessary.

In cohort 1, no patients treated with 150 mg SCO-101 and having a RAS mutated cancer have continued in the study after the initial 4 treatment cycles. All patients have at this point either been withdrawn from study due to poor health condition or progression of disease.

In cohort 2 (100 mg SCO-101), two patients have been treated with 100 mg SCO-101 and 50% FOLFIRI (of the standard recommended dose) and have tolerated the combination well without developing grade 3 or grade 4 neutropenia or other dose limiting toxicities. Thus, patients having a RAS mutated cancer require a different treatment regimen than patients having a cancer which is not RAS mutated.

### Conclusion

The present example demonstrates that patients with a RAS mutated cancer, require a lowered dosage of the combination treatment of SCO-101 and FOLFIRI compared to the patients having cancer without a RAS mutation, and further that the patients with the RAS mutated cancer tolerate the lowered dosage well.

### Example 4: Identification of treatment regimen with SCO-101 and an anti-cancer agent by RAS mutation status

### Materials & methods

This example is based on the clinical study of Example 1.

### Results

As is seen in the Kaplan-Meyer plot on Fig.1, there is a clear correlation between RAS mutation status and the cumulative progression free survival. Patient having a RAS mutated cancer do not tolerate the same dosage of SCO-101 and FOLFIRI as the patients having RAS wild type cancer. The patients having a RAS mutated cancer have increased risk of neutropenia. RAS mutation status can thus be used as indicator of patients' tolerability to the treatment and be used to determine the treatment regimen, Fig. 3.

### Conclusion

The present example demonstrates that by examining the RAS mutation status prior to initiating treatment using SCO-101 and an anti-cancer agent, an improved treatment regimen can be deduced prior to treatment. Thus, a method employing a predetermination of RAS mutation status prior to treatment can improve the course of treatment for patients about to receive combination therapy by limiting the risk of severe side effects, e.g. neutropenia, and the need for later dose adjustments and associated risk of treatment delays and disease progression.

### Example 5: Identification of tolerable anti-cancer agent dosage by pre-examination with SCO-101

### Materials & methods

This example is based on the clinical study of Example 1.

### Results

SCO-101 is observed to cause a specific and dose dependent increase in unconjugated bilirubin serum concentration and the effect is reversible following discontinuation of SCO-101 treatment. Likely, the increase in unconjugated bilirubin is mediated through inhibition of the enzyme UDP-glucuronosyltransferase 1 family, polypeptide A1 (UGT1A1). UGT1A1 is an enzyme responsible for conjugation of poorly soluble endo- and xenobiotics making them more soluble and possible to excrete. UGT1A1 is the primary enzyme responsible for conjugation of bilirubin and metabolism of Irinotecan's active metabolite SN-38. The bilirubin index score is the bilirubin serum concentration (unconjugated or total bilirubin) on day 12 divided with the bilirubin serum concentration on day 7 and thus is an indicator of how much the patients' UGT1A1 activity has normalized following cessation of SCO-101 treatment, and can be determined using bilirubin as a surrogate marker. In Fig. 2, an overview of the bilirubin index for all patients is included. The mean bilirubin index score for the six RAS wild type patients is approximately 0.15 (lowest 0.04, highest 0.28) whereas the mean score for the six RAS mutated patients is 0.59 (lowest 0.25, highest 0.76). Thus, there is a strong indication that the bilirubin index can predict a patient's tolerability to treatment. RAS mutated cancers appear to result in an impaired ability to normalize UGT1A1 activity following exposure to SCO-101 and thus an increased risk of developing grade 3 and grade 4 neutropenia.

### Conclusion

The present example demonstrates that by administering SCO-101 prior to initiating cancer therapy, and measuring the rate of unconjugated bilirubin normalization i.e. the bilirubin index, a more precise anti-cancer agent dosage can be determined which is less likely to induce side effects. Thus, a method employing a pre-administration of SCO-101 followed by determination of the bilirubin index prior to administration of anti-cancer agents can improve the course of treatment for patients about to receive cancer therapy with an UGT1A1 substrate in combination with an UGT1A1 inhibitor such as SCO-101.

### Example 6: Predicting if a patient is at risk of developing grade 3 or 4 neutropenia by pre-examination with SCO-101

### Materials & methods

This example is based on the clinical study of Example 1.

### Results

SCO-101 is observed to cause a specific and dose dependent increase in unconjugated bilirubin serum concentration and the effect is reversible following discontinuation of SCO-101 treatment. Likely, the increase in unconjugated bilirubin is mediated through SCO-101-mediated inhibition of the enzyme UDP-glucuronosyltransferase 1 family, polypeptide A1 (UGT1A1). UGT1A1 is an enzyme responsible for conjugation of poorly soluble endo- and xenobiotics making them more soluble and possible to excrete. UGT1A1 is the primary enzyme responsible for conjugation of bilirubin and metabolism of Irinotecan's active metabolite SN-38. The bilirubin index score is the bilirubin serum concentration (unconjugated or total bilirubin) on day 12 divided with the bilirubin serum concentration on day 7 and thus is an indicator of how much the patients' UGT1A1 activity has normalized following cessation of SCO-101 treatment, and can be determined using bilirubin as a surrogate marker. In Fig. 2, an overview of the bilirubin index for all patients is included. The mean bilirubin index score for the six RAS wild type patients is approximately 0.15 (lowest 0.04, highest 0.28) whereas the mean score for the six RAS mutated patients is 0.59 (lowest 0.25, highest 0.76). Thus, there is a strong indication that the bilirubin index can predict a patient's tolerability to treatment. RAS mutated cancers appear to result in an impaired ability to normalize UGT1A1 activity following exposure to SCO-101 and thus an increased risk of developing grade 3 and grade 4 neutropenia.

### Conclusion

The present example demonstrates that by administering SCO-101 prior to initiating cancer therapy, and measuring the rate of unconjugated bilirubin normalization i.e. the bilirubin index, patients can be identified which are at increased risk of developing grade 3 or 4 neutropenia from cancer therapy, in particular from treatments comprising an UGT1A1 substrate, such as irinotecan and an UGT1A1 inhibitor, such as SCO-101.

## Claims

1. A combination drug comprising, separately or together, an UGT1A1 inhibitor and an anti-cancer agent for use in the treatment of cancer in a patient; wherein the cancer is not a RAS mutated cancer; wherein the anti-cancer agent is an UGT1A1 substrate; and wherein the UGT1A1 inhibitor is SCO-101: 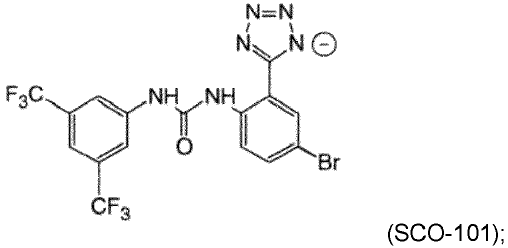 or a pharmaceutically acceptable salt thereof.

2. A combination drug comprising, separately or together, an UGT1A1 inhibitor and an anti-cancer agent for use in the treatment of cancer in a patient; wherein the cancer is a RAS wild-type; wherein the anti-cancer agent is an UGT1A1 substrate; and wherein the UGT1A1 inhibitor is SCO-101: 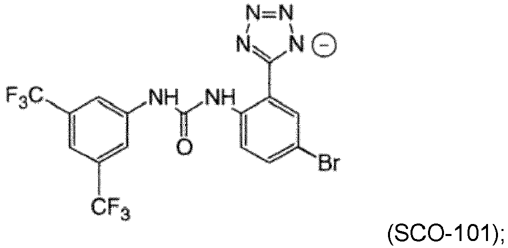 or a pharmaceutically acceptable salt thereof.

3. The combination drug for use according to any one of the preceding claims,
wherein SCO-101 is administered to the patient in a daily dosage of 0.3 mmol.

4. The combination drug for use according to any one of the preceding claims,
wherein SCO-101 is administered to the patient in a daily dosage of 0.3 mmol; and the anti-cancer agent is administered to the patient at from 40% to 60% of the recommended dose according to the Summary of Product Characteristics, such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60%.

5. The combination drug for use according to any one of the preceding claims,
wherein SCO-101 is administered to the patient in a daily dosage of 0.3 mmol; and the anti-cancer agent is irinotecan which is administered to the patient at from 40% to 60% of 180 mg/m², such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60% of 180 mg/m².

6. A combination drug comprising, separately or together, an UGT1A1 inhibitor and an anti-cancer agent for use in the treatment of cancer in a patient; wherein the cancer is a RAS mutated cancer; wherein the anti-cancer agent is an UGT1A1 substrate; and wherein the UGT1A1 inhibitor is SCO-101: 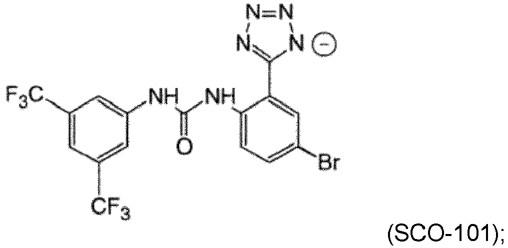 or a pharmaceutically acceptable salt thereof.

7. The combination drug for use according to claim 6, wherein SCO-101 is administered to the patient in a daily dosage of 0.2 mmol.

8. The combination drug for use according to any one of claims 6-7, wherein SCO-101 is administered to the patient in a daily dosage of 0.2 mmol; and the anti-cancer agent is irinotecan which is administered to the patient at from 40% to 60% of 180 mg/m², such as from 40 to 41%, such as from 41% to 42%, such as from 42% to 43%, such as from 43% to 44%, such as from 44% to 45%, such as from 45% to 46%, such as from 46% to 47%, such as from 47% to 48%, such as from 48% to 49%, such as from 49% to 50%, such as from 50% to 51%, such as from 51% to 52%, such as from 52% to 53%, such as from 53% to 54%, such as from 54% to 55%, such as from 55% to 56%, such as from 56% to 57%, such as from 57% to 58%, such as from 58% to 59%, such as from 59% to 60% of 180 mg/m².

9. The combination drug for use according to any one of claims 1-7, wherein the anti-cancer agent is a topoisomerase I inhibitor selected from the group consisting of: irinotecan, its active metabolite SN-38, and topotecan.

10. A method for identifying a treatment regimen for a patient having cancer with an UGT1A1 inhibitor and an anti-cancer agent; said method comprising determining if the patient has a RAS mutated cancer; and
wherein if the cancer is not a RAS mutated cancer, the dosage regimen is a first predefined dosage of the UGT1A1 inhibitor and a first predefined dosage of the anti-cancer agent; or
wherein if the cancer is a RAS mutated cancer, the dosage regimen is a second predefined dosage of the UGT1A1 inhibitor and a second predefined dosage of the anti-cancer agent;
wherein the first and second predefined dosages of the anti-cancer agent are different; and/or
wherein the first and second predefined dosages of the UGT1A1 inhibitor are different; and
wherein the anti-cancer agent is an UGT1A1 substrate; and wherein the UGT1A1 inhibitor is SCO-101: 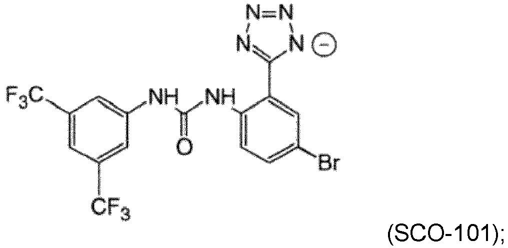 or a pharmaceutically acceptable salt thereof.

11. The method according to claim 10, wherein the first and second predefined dosages of the anti-cancer agent are the same; and the first predefined dosage of the UGT1A1 inhibitor is higher than the second predefined dosage of the UGT1A1 inhibitor.

12. A method for selecting an anti-cancer agent dosage regimen for a cancer patient, wherein the anti-cancer agent is an UGT1A1 substrate; said method comprising the consecutive steps of:
1) administering a first predefined dosage of an UGT1A1 inhibitor to the patient one or more times;
2) determining the plasma concentration of unconjugated bilirubin in the patient at a first time point;
3) discontinuing administration of the UGT1A1 inhibitor;
4) determining the plasma concentration of unconjugated bilirubin at a second time point which is subsequent in time to the first time point;
5) generating a bilirubin index being the second plasma concentration of unconjugated bilirubin divided by the first plasma concentration of unconjugated bilirubin, and
wherein if the bilirubin index is below a threshold value, selecting a first predefined dosage of the anti-cancer agent as dosage regimen; or
wherein if the bilirubin index is above a threshold value, selecting a second predefined dosage of the anti-cancer agent as dosage regimen;
wherein the first and second predefined dosages of the anti-cancer agent are different; and
wherein the UGT1A1 inhibitor is SCO-101: 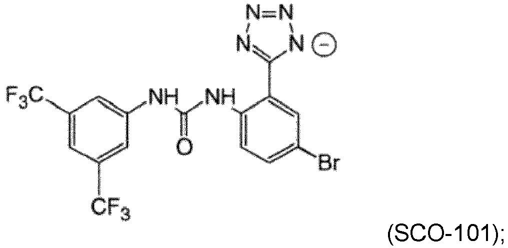 or a pharmaceutically acceptable salt thereof.

13. A method for predicting if a patient has or is likely to have a RAS-like mutated cancer, wherein the anti-cancer agent is an UGT1A1 substrate, said method comprising the consecutive steps of:
1) administering a first predefined dosage of an UGT1A1 inhibitor to the patient one or more times; and
2) determining a first concentration of unconjugated bilirubin at a first time point;
3) discontinuing treatment with the UGT1A1 inhibitor;
4) determining a second concentration of unconjugated bilirubin at a second time point which is subsequent in time to the first time point; and
wherein the patient has or is likely to have a RAS-like mutated cancer if the bilirubin index being the second concentration divided by the first concentration is above a threshold value; and/or
wherein the patient does not have or is not likely to have a RAS-like mutated cancer if the bilirubin index being the second concentration divided by the first concentration is below the threshold value; and
wherein the UGT1A1 inhibitor is SCO-101: 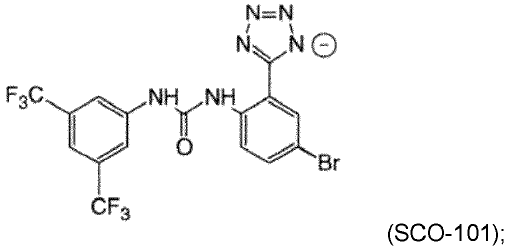 or a pharmaceutically acceptable salt thereof.

14. The method according to any one of claims 10-13, wherein the anti-cancer agent is a topoisomerase I inhibitor selected from the group consisting of: irinotecan, its active metabolite SN-38, and topotecan.

15. A combination drug comprising,
a) separately or together, 0.3 mmol of an UGT1A1 inhibitor, and 50% of 180 mg/m² of an anti-cancer agent which is an UGT1A1 substrate; or
b) separately or together, 0.2 mmol of an UGT1A1 inhibitor, and 50% of 180 mg/m² of an anti-cancer agent which is an UGT1A1 substrate;
wherein the UGT1A1 inhibitor is SCO-101: 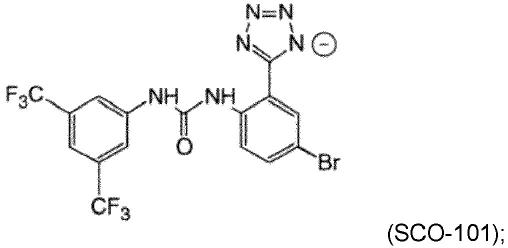 or a pharmaceutically acceptable salt thereof.
